# EUROPÄISCHE PATENTANMELDUNG

(11) **EP 1 780 206 A1**
(43) Veröffentlichungstag der Anmeldung: **02.05.2007**
(21) Anmeldenummer: 05023584.5
(22) Anmeldetag: 28.10.2005
(51) Int. Cl.: C07D 401/04, C07D 403/04, A01N 43/56

(54) **Verwendung von 5-Amino-pyrazolen zur Bekaempfung pflanzenpathogener Schadpilze, neue 5-Amino-pyrazole, Verfahren zu ihrer Herstellung und sie enthaltende Mittel**

(71) Anmelder: BASF Aktiengesellschaft, 67056 Ludwigshafen (DE)
(72) Erfinder: Hünger, Udo Dr., 68167 Mannheim (DE)

(57) **Zusammenfassung**

Verwendung von 5-Amino-pyrazole der Formel I 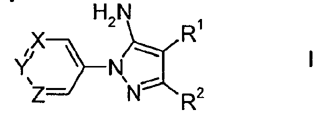 in der die Substituenten folgende Bedeutung haben:
R¹
Halogen, Alkyl, Halogenalkyl, Cycloalkyl, Halogencycloalkyl, Alkenyl, Halogenalkenyl, Alkinyl, Alkoxyalkyl, Halogenalkoxyalkyl, Alkoxyhalogenalkyl, Cyanoalkyl, Alkylalkoxyimino, Phenyl, Benzyloxyalkyl, oder ein fünf- oder sechsgliedriger gesättigter, partiell ungesättigter oder aromatischer Heterocyclus, enthaltend ein bis vier Heteroatome aus der Gruppe O, N oder S, welcher entweder direkt oder über eine Alkylengruppe gebunden sind;
R²
Halogen, Cyano, Alkyl, Halogenalkyl, Alkenyl, Alkinyl, Cycloalkyl, Alkoxy, C₁-C₁₂-Alkoxyalkyl, Alkylalkoxyimino, Phenyl, Benzyloxyalkyl, oder ein fünf- oder sechsgliedriger gesättigter, partiell ungesättigter oder aromatischer Heterocyclus, enthaltend ein bis vier Heteroatome aus der Gruppe O, N oder S, welcher entweder direkt oder über eine Alkylengruppe gebunden sind;
X, Y, Z
X oder Y oder X und Z oder Y und Z für N und die jeweiligen anderen Variablen für C-R³;
R³
Wasserstoff oder Halogen, Cyano, Hydroxy, Mercapto, Alkyl, Halogenalkyl, Cycloalkyl, Alkenyl, Alkinyl, Alkoxy, Alkylthio, Alkoxyalkyl, NR^{A}R^{B}, Phenyl, Alkyl-phenyl;;

die Gruppen R¹, R² und R³ können unsubstituiert oder gemäß der Beschreibung substituiert sein;
zur Bekämpfung von pflanzenpathogenen Schadpilzen;
neue 5-Amino-pyrazole, Verfahren zur Herstellung dieser Verbindungen sowie sie enthaltende Mittel.

## Beschreibung

Die vorliegende Erfindung betrifft die Verwendung von 5-Amino-pyrazole der Formel I 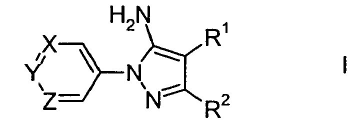 in der die Substituenten folgende Bedeutung haben:
- R¹: Halogen, C₁-C₁₂-Alkyl, C₁-C₁₂-Halogenalkyl, C₃-C₆-Cycloalkyl, C₃-C₆-Halogen-cycloalkyl, C₂-C₁₂-Alkenyl, C₂-C₁₂-Halogenalkenyl, C₂-C₁₂-Alkinyl, C₂-C₁₂-Alkoxy-alkyl, C₂-C₁₂-Halogenalkoxyalkyl, C₂-C₁₂-Alkoxyhalogenalkyl, C₂-C₁₂-Cyanoalkyl, C₁-C₁₂-Alkyl-C₁-C₁₂-alkoxyimino, Phenyl, Benzyloxy-C₁-C₁₂-alkyl, oder ein fünfoder sechsgliedriger gesättigter, partiell ungesättigter oder aromatischer Hetero-cyclus, enthaltend ein bis vier Heteroatome aus der Gruppe O, N oder S, welcher entweder direkt oder über eine C₁-C₄-Alkylengruppe gebunden sind;
- R²: Halogen, Cyano, C₁-C₁₂-Alkyl, C₁-C₁₂-Halogenalkyl, C₂-C₁₂-Alkenyl, C₂-C₁₂ Alkinyl, C₃-C₈-Cycloalkyl, C₁-C₁₂-Alkoxy, C₁-C₁₂-Alkoxy-C₁-C₁₂-alkyl, C₁-C₁₂-Alkyl-C₁-C₁₂-alkoxyimino, Phenyl, Benzyloxy-C₁-C₁₂-alkyl, oder ein fünf- oder sechsgliedriger gesättigter, partiell ungesättigter oder aromatischer Heterocyclus, enthaltend ein bis vier Heteroatome aus der Gruppe O, N oder S, welcher entweder direkt oder über eine C₁-C₄-Alkylengruppe gebunden sind;
- X, Y, Z: X oder Y oder X und Z oder Y und Z für N und die jeweiligen anderen Variablen für C-R³;
R³ Wasserstoff oder eine der bei R^{a} genannten Gruppen; die Gruppen R¹, R² und R³ können unabhängig voneinander unsubstituiert oder durch eine bis fünf Gruppen R^{a} substituiert sein:
R^{a} Halogen, Cyano, Hydroxy, Mercapto, C₁-C₁₀-Alkyl, C₁-C₁₀-Halogenalkyl, C₃-C₈-Cycloalkyl, C₂-C₁₀-Alkenyl, C₂-C₁₀-Alkinyl, C₁-C₆-Alkoxy, C₁-C₆-Alkylthio, C₁-C₆-Alkoxy-C₁-C₆-alkyl, NR^{A}R^{B}, Phenyl, C₁-C₆-Alkyl-phenyl;
R^{A}, R^{B} Wasserstoff und C₁-C₆-Alkyl; wobei die cyclischen Gruppen in R^{a} durch eine bis vier Gruppen R^{b} substituiert sein können:
R^{b} Halogen, Cyano, Hydroxy, Mercapto, C₁-C₁₀-Alkyl, C₁-C₁₀-Halogenalkyl, C₂-C₁₀-Alkenyl, C₂-C₁₀-Alkinyl und C₁-C₆-Alkoxy;
zur Bekämpfung von pflanzenpathogenen Schadpilzen.

Außerdem betrifft die Erfindung neue 5-Amino-pyrazole, Verfahren zur Herstellung dieser Verbindungen sowie sie enthaltende Mittel.

Aus DE-OS 195 18 054, EP-A 293 743 und JP-A 8-208 620 sind fungizid wirksame 5-Amino-pyrazole bekannt. Ihre Wirkung ist jedoch in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäß wurde die Verwendung der eingangs definierten Verbindungen gefunden. Des weiteren wurden neue 5-Amino-pyrazole, Verfahren und Zwischenprodukte zu ihrer Herstellung sowie sie enthaltende Mittel gefunden.

Die Verbindungen der Formel I weisen eine gegenüber den bekannten fungiziden Verbindungen erhöhte Wirksamkeit gegen Schadpilze auf.

Die erfindungsgemäßen Verbindungen können auf verschiedenen Wegen nach an sich literaturbekannten Verfahren erhalten werden. Vorteilhaft werden sie erhalten, indem man substituierte α-Ketonitrile der Formel II mit Hydrazinen der Formel III umsetzt. Die Gruppen R¹ und R² in Formeln II und III haben die Bedeutungen wie für Formel I. 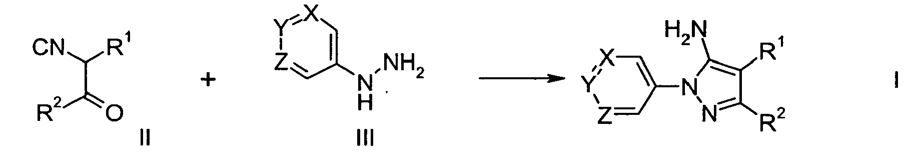 Die Umsetzung kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, aromatische Kohlenwasserstoffe, wie Toluol, Benzol oder Mesitylen, Amide wie Dimethylformamid, Diethylformamid, Dibutylformamid, N,N-Dimethylacetamid, niedere Alkansäuren wie Ameisensäure, Essigsäure, Propionsäure oder Basen, wie voranstehend genannt, und Mischungen dieser Lösungsmittel mit Wasser in Frage. Die Umsetzungstemperaturen liegen zwischen 50 und 300°C, vorzugsweise bei 50 bis 150°C, wenn in Lösung gearbeitet wird.

Alternativ können die Hydrazine mit β-Ketoestern der Formel IIa umgesetzt werden, die entstehenden Verbindungen IIb werden nach Halogenierung und Amidierung zu den Verbindungen der Formel I cyclisiert. 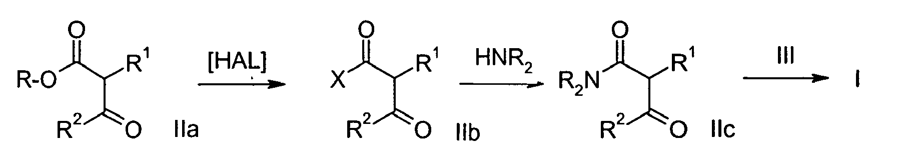 In Formeln IIa, HNR₂ und IIc bedeutet die Gruppe R C₁-C₄-Alkyl, aus praktischen Gründen ist Methyl, Ethyl oder Propyl darin bevorzugt. β-Ketoestern der Formel IIa werden mit Halogenierungsmitteln, wie Chlorierungs- oder Bromierungsmitteln, zu den Säurechloriden der Formel IIb, in der Hal für Halogen, wie Chlor oder Brom, insbesondere für Chlor steht, umgesetzt. Bevorzugt erfolgt die Umsetzung mit Chlorierungsmitteln, wie Phosphoroxychlorid, Thionylchlorid oder Sulfurylchlorid bei 50°C bis 150°C vorzugsweise in überschüssigem Phosphoroxitrichlorid bei Rückflusstemperatur.

Die Umsetzung der Säurechloride IIb mit den Aminen HNR₂ erfolgt unter an sich bekannten Bedingungen.

Die Cyclisierung der Verbindungen IIc mit Hydrazinen III verläuft unter den weiter oben geschilderten Bedingungen.

Die substituierten α-Ketonitrile der Formel II sind teilweise bekannt oder können nach bekannten Methoden aus Alkylcyaniden und Carbonsäureestern mit starken Basen, z.B. Alkalihydriden, Alkalimetallalkoholaten, Alkaliamiden oder Metallalkylen, hergestellt werden [vgl.: J. Amer. Chem. Soc. Bd. 73, (1951) S. 3766]. Die β-Ketoester der Formel IIa können hergestellt werden wie in Organic Synthesis Coll. Vol. 1, S. 248 beschrieben, bzw. sind kommerziell erhältlich.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säureoder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 2, 4 oder 6 Kohlenstoffatomen (wie vorstehend genannt), wobei in diesen Gruppen teilweise oder vollständig die Wasserstoffatome durch Halogenatome wie vorstehend genannt ersetzt sein können: insbesondere C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
Alkenyl: ungesättigte, geradkettige oder verzweigte Kohlenwasserstoffreste mit 2 bis 4, 6, 8 oder 10 Kohlenstoffatomen und einer oder zwei Doppelbindungen in beliebiger Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Halogenalkenyl: ungesättigte, geradkettige oder verzweigte Kohlenwasserstoffreste mit 2 bis 10 Kohlenstoffatomen und einer oder zwei Doppelbindungen in beliebiger Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
Alkinyl: geradkettige oder verzweigte Kohlenwasserstoffgruppen mit 2 bis 4, 6, 8 oder 10 Kohlenstoffatomen und einer oder zwei Dreifachbindungen in beliebiger Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Cycloalkyl: mono- oder bicyclische, gesättigte Kohlenwasserstoffgruppen mit 3 bis 6 Kohlenstoffringgliedern, wie Cyclopropyl, Cyclobutyl, Cyclopentyl und Cyclohexyl;
Alkoxyalkyl: gesättigte, geradkettige oder ein-, zwei- oder dreifach verzweigte Kohlenwasserstoffkette, die durch ein Sauerstoffatom unterbrochen ist, z. B. C₅-C₁₂-Alkoxyalkyl: Kohlenwasserstoffkette wie voranstehend beschreiben mit 5 bis 12 Kohlenstoffatomen, die durch ein Sauerstoffatom an beliebiger Stelle unterbrochen sein kann, wie Propoxy-ethyl, Butoxy-ethyl, Pentoxy-ethyl, Hexyloxy-ethyl, Heptyloxy-ethyl, Octyloxyethyl, Nonyloxy-ethyl, 3-(3-Ethyl-hexyloxy)-ethyl, 3-(2,4,4-Trimethyl-pentyloxy)-ethyl, 3-(1-Ethyl-3-methyl-butoxy)-ethyl, Ethoxy-propyl, Propoxy-propyl, Butoxy-propyl, Pentoxy-propyl, Hexyloxy-propyl, Heptyloxy-propyl, Octyloxy-propyl, Nonyloxy-propyl, 3-(3-Ethyl-hexyloxy)-propyl, 3-(2,4,4-Trimethyl-pentyloxy)-propyl, 3-(1-Ethyl-3-methyl-butoxy)-propyl, Ethoxy-butyl, Propoxy-butyl, Butoxy-butyl, Pentoxy-butyl, Hexyloxybutyl, Heptyloxy-butyl, Octyloxy-butyl, Nonyloxy-butyl, 3-(3-Ethyl-hexyloxy)-butyl, 3-(2,4,4-Trimethyl-pentyloxy)-butyl, 3-(1-Ethyl-3-methyl-butoxy)-butyl, Methoxy-pentyl, Ethoxy-pentyl, Propoxy-pentyl, Butoxy-pentyl, Pentoxy-pentyl, Hexyloxy-pentyl, Heptyloxy-pentyl, 3-(3-Methyl-hexyloxy)-pentyl, 3-(2,4-Dimethyl-pentyloxy)-pentyl, 3-(1-Ethyl-3-methyl-butoxy)-pentyl;

Fünf- oder sechsgliedriger gesättigter, partiell ungesättigter oder aromatischer Heterocyclus, enthaltend ein bis vier Heteroatome aus der Gruppe O, N oder S:
- 5- oder 6-gliedriges Heterocyclyl, enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
- 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl.

In dem Umfang der vorliegenden Erfindung sind die (R)- und (S)-Isomere und die Razemate von Verbindungen der Formel I eingeschlossen, die chirale Zentren aufweisen.

Im Hinblick auf ihre bestimmungsgemäße Verwendung der 5-Aminopyrazole der Formel I sind die folgenden Bedeutungen der Substituenten, und zwar jeweils für sich allein oder in Kombination, besonders bevorzugt:

Verbindungen I werden bevorzugt, in denen die Gruppe R¹ maximal 12 Kohlenstoffatome aufweist.

Die Alkylgruppen in R¹ in Formel I stellen bevorzugt unverzweigte oder ein-, zwei-, dreioder mehrfach verzweigte, insbesondere eine unverzweigte Alkylgruppe dar.

Sofern R¹ eine durch Cyano substituierte Alkylgruppe darstellt, steht die Cyanogruppe bevorzugt am endständigen Kohlenstoffatom.

Sofern R¹ eine durch Halogen substituierte Alkylgruppe darstellt, liegt die Halogenierung bevorzugt am α- oder am Ω-Kohlenstoffatom vor.

In einer weiteren Ausgestaltung steht R¹ für eine durch Hydroxy substituierte Alkylgruppe.

Bevorzugt sind Verbindungen I, in denen R¹ für eine unverzweigte oder ein-, zwei-, drei- oder mehrfach verzweigte C₅-C₁₂-Alkylgruppe oder C₁-C₁₁-Alkoxy-C₁-C₁₂-alkyl mit fünf bis 12 Kohlenstoffatomen steht. Besonders bevorzugt sind Verbindungen I, in denen R¹ für eine C₅-C₁₂-Alkylgruppe steht.

In einer weiteren Ausführungsform der erfindungsgemäßen Verbindungen I, in denen R¹ für C₂-C₁₂-Alkenyl steht, insbesondere C₅-C₁₂-Alkenyl.

In einer weiteren Ausführungsform der erfindungsgemäßen Verbindungen I, in denen R¹ für C₂-C₁₂-Alkinyl steht, insbesondere C₅-C₁₂-Alkinyl.

In einer weiteren Ausführungsform der erfindungsgemäßen Verbindungen I, in denen R¹ für Phenyl, Benzyl oder Phenethyl steht, welche im Phenylteil durch eine bis fünf Gruppen R^{a} substituiert sein können, insbesondere für ein- oder zweifach substituierte, wobei die Substituenten in meta- oder para-Position stehen. Diese Phenyl, Benzyl oder Phenethylgruppen entsprechen der Gruppe G, worin die Gruppen L¹ bis L⁵ unabhängig voneinander für Wasserstoff oder einer Gruppe R^{a} entsprechen und # die direkte Bindung, die Methylen- oder Ethylengruppe zum Pyrazolring darstellt. 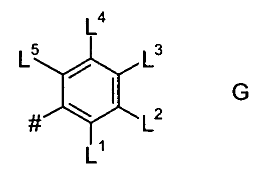 In einer weiteren Ausführungsform der erfindungsgemäßen Verbindungen I steht R¹ für n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl steht.

In einer weiteren Ausführungsform der erfindungsgemäßen Verbindungen I steht R¹ für n-Heptyl, 1-Methylhexyl, n-Octyl, 1-Methylheptyl, n-Nonyl, 1-Methyloctyl, 3,5,5-Trimethylhexyl, n-Decyl, 1-Methylnonyl, n-Undecyl, 1-Methyldecyl, n-Dodecyl und 1-Methylundecyl steht.

In einer weiteren Ausführungsform der erfindungsgemäßen Verbindungen I steht R² für Alkyl, Alkenyl, Alkoxyalkyl oder Alkinyl, welche Gruppen bis zu fünf Kohlenstoffatome aufweisen.

In einer bevorzugten Ausführungsform der erfindungsgemäßen Verbindungen I steht R² für C₁-C₅-Alkyl.

In einer weiteren Ausführungsform der erfindungsgemäßen Verbindungen 1 steht R² für C₃-C₅-Alkenyl.

In einer weiteren Ausführungsform der erfindungsgemäßen Verbindungen I steht R² für C₃-C₅-Alkinyl.

Besonders bevorzugt sind Verbindungen I, in denen R² für C₁-C₃-Alkyl oder C₁-C₃-Alkoxy-C₁-C₃-Alkyl, insbesondere für Methyl, Ethyl, n-Propyl oder Methoxymethyl steht.

In einer anderen bevorzugten Ausgestaltung der erfindungsgemäßen Verbindungen I steht R² für C₁-C₅-Halogenalkyl, insbesondere für Halogenmethyl, wie Trifluormethyl.

In einer anderen bevorzugten Ausgestaltung der erfindungsgemäßen Verbindungen I steht R² für C₁-C₅-Alkoxy-C₁-C₅-alkyl, insbesondere für Alkoxymethyl, wie Methoxymethyl.

In einer Ausgestaltung der erfindungsgemäßen Verbindungen I steht R³ für einen heterocyclischen Ring ausgewählt aus der folgenden Gruppe: 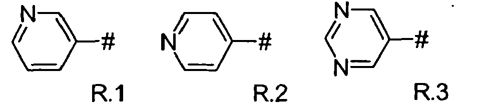 welcher durch eine, zwei oder drei gleiche oder verschiedene Gruppen R^{a} substituiert sein kann, wobei # die Bindung zu dem zentralen Pyrazolgerüst kennzeichnet.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel 1.1, in denen R¹ für eine Verbindung jeweils einer Zeile der Tabelle A entspricht und R² Methyl und R³ Wasserstoff bedeutet

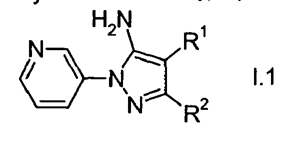

### Tabelle 2

Verbindungen der Formel 1.1, in denen R¹ für eine Verbindung jeweils einer Zeile der Tabelle A entspricht und R² Ethyl und R³ Wasserstoff bedeutet

### Tabelle 3

Verbindungen der Formel I.1, in denen R¹ für eine Verbindung jeweils einer Zeile der Tabelle A entspricht und R² n-Propyl und R³ Wasserstoff bedeutet

### Tabelle 4

Verbindungen der Formel I.1, in denen R¹ für eine Verbindung jeweils einer Zeile der Tabelle A entspricht und R² Methoxymethyl und R³ Wasserstoff bedeutet

### Tabelle 5

Verbindungen der Formel I.1, in denen R¹ für eine Verbindung jeweils einer Zeile der Tabelle A entspricht und R² Ethoxymethyl und R³ Wasserstoff bedeutet.

### Tabelle 6

Verbindungen der Formel I.1, in denen R¹ für eine Verbindung jeweils einer Zeile der Tabelle A entspricht und R² n-Propoxymethyl und R³ Wasserstoff bedeutet.

### Tabelle 7

Verbindungen der Formel I.1, in denen R¹ für eine Verbindung jeweils einer Zeile der Tabelle A entspricht und R² Methoxyethyl und R³ Wasserstoff bedeutet.

### Tabelle 8

Verbindungen der Formel I.1, in denen R¹ für eine Verbindung jeweils einer Zeile der Tabelle A entspricht und R² Ethoxyethyl und R³ Wasserstoff bedeutet.

### Tabelle 9

Verbindungen der Formel I.1, in denen R¹ für eine Verbindung jeweils einer Zeile der Tabelle A entspricht und R² n-Propoxyethyl und R³ Wasserstoff bedeutet.

### Tabelle 10

Verbindungen der Formel I.1, in denen R¹ für eine Verbindung jeweils einer Zeile der Tabelle A entspricht und R² 3-Methoxy-n-propyl.und R³ Wasserstoff bedeutet.

### Tabelle 11

Verbindungen der Formel I.1, in denen R¹ für eine Verbindung jeweils einer Zeile der Tabelle A entspricht und R² 3-Ethoxy-n-propyl und R³ Wasserstoff bedeutet.

### Tabelle 12

Verbindungen der Formel I.1, in denen R¹ für eine Verbindung jeweils einer Zeile der Tabelle A entspricht und R² 3-n-Propoxy-n-propyl und R³ Wasserstoff bedeutet.

### Tabelle 13

Verbindungen der Formel I.1, in denen R¹ für eine Verbindung jeweils einer Zeile der Tabelle A entspricht und R² Ethenyl und R³ Wasserstoff bedeutet

### Tabelle 14

Verbindungen der Formel 1.1, in denen R¹ für eine Verbindung jeweils einer Zeile der Tabelle A entspricht und R² Allyl und R³ Wasserstoff bedeutet

### Tabelle 15

Verbindungen der Formel I.1, in denen R¹ für eine Verbindung jeweils einer Zeile der Tabelle A entspricht und R² Trifluormethyl und R³ Wasserstoff bedeutet.

### Tabelle 16

Verbindungen der Formel 1.2, in denen R¹ für eine Verbindung jeweils einer Zeile der Tabelle A entspricht und R² Methyl und R³ Wasserstoff bedeutet

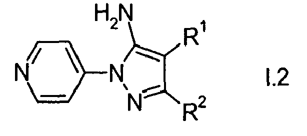

### Tabelle 17

Verbindungen der Formel 1.2, in denen R¹ für eine Verbindung jeweils einer Zeile der Tabelle A entspricht und R² Ethyl und R³ Wasserstoff bedeutet

### Tabelle 18

Verbindungen der Formel 1.2, in denen R¹ für eine Verbindung jeweils einer Zeile der Tabelle A entspricht und R² n-Propyl und R³ Wasserstoff bedeutet

### Tabelle 19

Verbindungen der Formel 1.2, in denen R¹ für eine Verbindung jeweils einer Zeile der Tabelle A entspricht und R² Methoxymethyl und R³ Wasserstoff bedeutet

### Tabelle 20

Verbindungen der Formel 1.2, in denen R¹ für eine Verbindung jeweils einer Zeile der Tabelle A entspricht und R² Ethoxymethyl und R³ Wasserstoff bedeutet.

### Tabelle 21

Verbindungen der Formel 1.2, in denen R¹ für eine Verbindung jeweils einer Zeile der Tabelle A entspricht und R² n-Propoxymethyl und R³ Wasserstoff bedeutet.

### Tabelle 22

Verbindungen der Formel I.2, in denen R¹ für eine Verbindung jeweils einer Zeile der Tabelle A entspricht und R² Methoxyethyl und R³ Wasserstoff bedeutet.

### Tabelle 23

Verbindungen der Formel I.2, in denen R¹ für eine Verbindung jeweils einer Zeile der Tabelle A entspricht und R² Ethoxyethyl und R³ Wasserstoff bedeutet.

### Tabelle 24

Verbindungen der Formel I.2, in denen R¹ für eine Verbindung jeweils einer Zeile der Tabelle A entspricht und R² n-Propoxyethyl und R³ Wasserstoff bedeutet.

### Tabelle 25

Verbindungen der Formel I.2, in denen R¹ für eine Verbindung jeweils einer Zeile der Tabelle A entspricht und R² 3-Methoxy-n-propyl und R³ Wasserstoff bedeutet.

### Tabelle 26

Verbindungen der Formel 1.2, in denen R¹ für eine Verbindung jeweils einer Zeile der Tabelle A entspricht und R² 3-Ethoxy-n-propyl und R³ Wasserstoff bedeutet.

### Tabelle 27

Verbindungen der Formel 1.2, in denen R¹ für eine Verbindung jeweils einer Zeile der Tabelle A entspricht und R² 3-n-Propoxy-n-propyl und R³ Wasserstoff bedeutet.

### Tabelle 28

Verbindungen der Formel 1.2, in denen R¹ für eine Verbindung jeweils einer Zeile der Tabelle A entspricht und R² Ethenyl und R³ Wasserstoff bedeutet

### Tabelle 29

Verbindungen der Formel 1.2, in denen R¹ für eine Verbindung jeweils einer Zeile der Tabelle A entspricht und R² Allyl und R³ Wasserstoff bedeutet

### Tabelle 30

Verbindungen der Formel I.2, in denen R¹ für eine Verbindung jeweils einer Zeile der Tabelle A entspricht und R² Trifluormethyl und R³ Wasserstoff bedeutet.

### Tabelle 31

Verbindungen der Formel 1.3, in denen R¹ für eine Verbindung jeweils einer Zeile der Tabelle A entspricht und R² Methyl und R³ Wasserstoff bedeutet

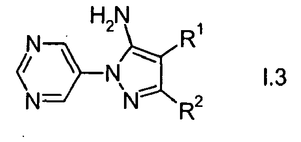

### Tabelle 32

Verbindungen der Formel I.3, in denen R¹ für eine Verbindung jeweils einer Zeile der Tabelle A entspricht und R² Ethyl und R³ Wasserstoff bedeutet

### Tabelle 33

Verbindungen der Formel I.3, in denen R¹ für eine Verbindung jeweils einer Zeile der Tabelle A entspricht und R² n-Propyl und R³ Wasserstoff bedeutet

### Tabelle 34

Verbindungen der Formel I.3, in denen R¹ für eine Verbindung jeweils einer Zeile der Tabelle A entspricht und R² Methoxymethyl und R³ Wasserstoff bedeutet

### Tabelle 35

Verbindungen der Formel I.3, in denen R¹ für eine Verbindung jeweils einer Zeile der Tabelle A entspricht und R² Ethoxymethyl und R³ Wasserstoff bedeutet.

### Tabelle 36

Verbindungen der Formel I.3, in denen R¹ für eine Verbindung jeweils einer Zeile der Tabelle A entspricht und R² n-Propoxymethyl und R³ Wasserstoff bedeutet.

### Tabelle 37

Verbindungen der Formel I.3, in denen R¹ für eine Verbindung jeweils einer Zeile der Tabelle A entspricht und R² Methoxyethyl und R³ Wasserstoff bedeutet.

### Tabelle 38

Verbindungen der Formel I.3, in denen R¹ für eine Verbindung jeweils einer Zeile der Tabelle A entspricht und R² Ethoxyethyl und R³ Wasserstoff bedeutet.

### Tabelle 39

Verbindungen der Formel I.3, in denen R¹ für eine Verbindung jeweils einer Zeile der Tabelle A entspricht und R² n-Propoxyethyl und R³ Wasserstoff bedeutet.

### Tabelle 40

Verbindungen der Formel I.3, in denen R¹ für eine Verbindung jeweils einer Zeile der Tabelle A entspricht und R² 3-Methoxy-n-propyl und R³ Wasserstoff bedeutet.

### Tabelle 41

Verbindungen der Formel I.3, in denen R¹ für eine Verbindung jeweils einer Zeile der Tabelle A entspricht und R² 3-Ethoxy-n-propyl und R³ Wasserstoff bedeutet.

### Tabelle 42

Verbindungen der Formel I.3, in denen R¹ für eine Verbindung jeweils einer Zeile der Tabelle A entspricht und R² 3-n-Propoxy-n-propyl und R³ Wasserstoff bedeutet.

### Tabelle 43

Verbindungen der Formel I.3, in denen R¹ für eine Verbindung jeweils einer Zeile der Tabelle A entspricht und R² Ethenyl und R³ Wasserstoff bedeutet

### Tabelle 44

Verbindungen der Formel I.3, in denen R¹ für eine Verbindung jeweils einer Zeile der Tabelle A entspricht und R² Allyl und R³ Wasserstoff bedeutet

### Tabelle 45

Verbindungen der Formel I.3, in denen R¹ für eine Verbindung jeweils einer Zeile der Tabelle A entspricht und R² Trifluormethyl und R³ Wasserstoff bedeutet.

**Tabelle A**

| **Nr.** | **R¹** |
|---|---|
| A-1 | CH₂CH₂CH₂CH₂CH₃ |
| A-2 | CH(CH₃)CH₂CH₂CH₃ |
| A-3 | CH₂CH(CH₃)CH₂CH₃ |
| A-4 | CH₂CH₂CH(CH₃)CH₃ |
| A-5 | CH₂CH₂CH(CH₃)₂ |
| A-6 | CH(CH₃)CH(CH₃)CH₃ |
| A-7 | CH(CH₃)CH(CH₃)₂ |
| A-8 | CH₂C(CH₃)₃ |
| A-9 | CH₂CH₂CH₂CH₂CH₂CH₃ |
| A-10 | CH(CH₃)CH₂CH₂CH₂CH₃ |
| A-11 | CH₂CH(CH₃)CH₂CH₂CH₃ |
| A-12 | CH₂CH₂CH(CH₃)CH₂CH₃ |
| A-13 | CH₂CH₂CH(CH₃)₂CH₂ |
| A-14 | CH₂CH₂CH₂CH(CH₃)₂ |
| A-15 | CH(CH₃)CH(CH₃)CH₂CH₃ |
| A-16 | CH(CH₃)CH₂CH(CH₃)₂ |
| A-17 | CH₂CH₂C(CH₃)₃ |
| A-18 | CH(CH₃)CH₂CH(CH₃)CH₃ |
| A-19 | CH₂CH₂CH₂CH₂CH₂CH₂CH₃ |
| A-20 | CH(CH₃)CH₂CH₂CH₂CH₂CH₃ |
| A-21 | CH₂CH(CH₃)CH₂CH₂CH₂CH₃ |
| A-22 | CH₂CH₂CH(CH₃)CH₂CH₂CH₃ |
| A-23 | CH₂CH₂CH₂CH(CH₃)CH₂CH₃ |
| A-24 | CH₂CH₂CH₂CH₂CH(CH₃)CH₃ |
| A-25 | CH₂CH₂CH₂CH₂CH(CH₃)₂ |
| A-26 | CH(CH₃)CH(CH₃)CH₂CH₂CH₃ |
| A-27 | CH₂CH(CH₃)CH(CH₃)CH₂CH₃ |
| A-28 | CH₂CH₂CH₂C(CH₃)₃ |
| A-29 | CH(CH₃)CH₂CH(CH₃)CH₂CH₃ |
| A-30 | CH₂CH(CH₃)CH(CH₃)CH₂CH₃ |
| A-31 | CH(CH₃)CH₂CH₂CH(CH₃)CH₃ |
| A-32 | CH₂CH₂CH₂CH₂CH₂CH₂CH₂CH₃ |
| A-33 | CH(CH₃)CH₂CH₂CH₂CH₂CH₂CH₃ |
| A-34 | CH₂CH(CH₃)CH₂CH₂CH₂CH₂CH₃ |
| A-35 | CH₂CH₂CH(CH₃)CH₂CH₂CH₂CH₃ |
| A-36 | CH₂CH₂CH₂CH(CH₃)CH₂CH₂CH₃ |
| A-37 | CH₂CH₂CH₂CH₂CH(CH₃)CH₂CH₃ |
| A-38 | CH₂CH₂CH₂CH₂CH₂CH(CH₃)₂ |
| A-39 | CH₂CH₂CH₂CH₂C(CH₃)₃ |
| A-40 | CH(CH₃)CH(CH₃)CH₂CH₂CH₂CH₃ |
| A-41 | CH₂CH(CH₃)CH(CH₃)CH₂CH₂CH₃ |
| A-42 | CH₂CH₂CH₂C(CH₃)₂CH₂CH₃ |
| A-43 | CH(CH₃)CH₂CH(CH₃)CH₂CH₂CH₃ |
| A-44 | CH₂CH(CH₃)CH(CH₃)CH₂CH₂CH₃ |
| A-45 | CH(CH₃)CH₂CH₂CH(CH₃)CH₂CH₃ |
| A-46 | CH(CH₃)CH₂CH₂CH₂CH(CH₃)₂ |
| A-47 | CH₂CH₂CH(CH₃)CH₂C(CH₃)₃ |
| A-48 | CH₂CH₂CH₂CH₂CH₂CH₂CH₂CH₂CH₃ |
| A-49 | CH(CH₃)CH₂CH₂CH₂CH₂CH₂CH₂CH₃ |
| A-50 | CH₂CH(CH₃)CH₂CH₂CH₂CH₂CH₂CH₃ |
| A-51 | CH₂CH₂CH(CH₃)CH₂CH₂CH₂CH₂CH₃ |
| A-52 | CH₂CH₂CH₂CH(CH₃)CH₂CH₂CH₂CH₃ |
| A-53 | CH₂CH₂CH₂CH₂CH(CH₃)CH₂CH₂CH₃ |
| A-54 | CH₂CH₂CH₂CH₂CH₂CH₂C(CH₃)₃ |
| A-55 | CH(CH₃)CH(CH₃)CH₂CH₂CH₂CH₂CH₃ |
| A-56 | CH₂CH(CH₃)CH(CH₃)CH₂CH₂CH₂CH₃ |
| A-57 | CH₂CH₂CH₂C(CH₃)₂CH₂CH₂CH₃ |
| A-58 | CH(CH₃)CH₂CH(CH₃)CH₂CH₂CH₂CH₃ |
| A-59 | CH₂CH(CH₃)CH(CH₃)CH₂CH₂CH₂CH₃ |
| A-60 | CH(CH₃)CH₂CH₂CH(CH₃)CH₂CH₂CH₃ |
| A-61 | CH(CH₃)CH₂CH₂CH₂C(CH₃)₃ |
| A-62 | CH₂CH(CH₃)CH₂CH₂CH(CH₃)₃ |
| A-63 | CH(CH₃)CH₂CH₂CH₂CH₂CH(CH₃)₂ |
| A-64 | CH₂CH(CH₃)CH₂CH₂CH₂CH(CH₃)₂ |
| A-65 | CH₂CH₂CH₂CH₂CH₂CH₂CH₂CH₂CH₂CH₃ |
| A-66 | CH(CH₃)CH₂CH₂CH₂CH₂CH₂CH₂CH₂CH₃ |
| A-67 | CH₂CH(CH₃)CH₂CH₂CH₂CH₂CH₂CH₂CH₃ |
| A-68 | CH₂CH₂CH(CH₃)CH₂CH₂CH₂CH₂CH₂CH₃ |
| A-69 | CH₂CH₂CH(CH₃)CH₂CH₂CH₂CH₂CH₂ |
| A-70 | CH₂CH₂CH₂CH(CH₃)CH₂CH₂CH₂CH₃ |
| A-71 | CH₂CH₂CH₂CH₂CH₂CH₂C(CH₃)₃ |
| A-72 | CH(CH₃)CH(CH₃)CH₂CH₂CH₂CH₂CH₂CH₃ |
| A-73 | CH₂CH(CH₃)CH(CH₃)CH₂CH₂CH₂CH₂CH₃ |
| A-74 | CH₂CH₂CH₂C(CH₃)₂CH₂CH₂CH₂CH₃ |
| A-75 | CH(CH₃)CH₂CH(CH₃)CH₂CH₂CH₂CH₂CH₃ |
| A-76 | CH₂CH(CH₃)CH(CH₃)CH₂CH₂CH₂CH₂CH₃ |
| A-77 | CH(CH₃)CH₂CH₂CH(CH₃)CH₂CH₂CH₂CH₃ |
| A-78 | CH(CH₃)CH₂CH₂CH₂CH(CH₃)CH₂CH₂CH₃ |
| A-79 | CH(CH₃)CH₂CH₂CH₂CH₂CH(CH₃)CH₂CH₃ |
| A-80 | CH(CH₃)CH₂CH₂CH₂CH₂CH₂CH(CH₃)₂ |
| A-81 | CH(CH₃)CH₂CH₂CH₂CH₂CH₂C(CH₃)CH₃ |
| A-82 | CH₂CH(CH₃)CH₂CH₂CH₂CH₂CH(CH₃)CH₃ |
| A-83 | CH(CH₃)CH₂CH₂CH₂CH₂C(CH₃)₃ |
| A-84 | CH₂CH(CH₃)CH₂CH₂CH₂C(CH₃)₃ |
| A-85 | CH₂CH₂CH₂CH₂CH₂CH₂CH₂CH₂CH₂CH₂CH₃ |
| A-86 | CH(CH₃)CH₂CH₂CH₂CH₂CH₂CH₂CH₂CH₂CH₃ |
| A-87 | CH₂CH(CH₃)CH₂CH₂CH₂CH₂CH₂CH₂CH₂CH₃ |
| A-88 | CH₂CH₂CH(CH₃)CH₂CH₂CH₂CH₂CH₂CH₂CH₃ |
| A-89 | CH₂CH₂CH₂CH(CH₃)CH₂CH₂CH₂CH₂CH₂CH₃ |
| A-90 | CH₂CH₂CH₂CH₂CH(CH₃)CH₂CH₂CH₂CH₂CH₃ |
| A-91 | CH₂CH₂CH₂CH₂CH₂CH₂CH₂C(CH₃)₃ |
| A-92 | CH(CH₃)CH(CH₃)CH₂CH₂CH₂CH₂CH₂CH₂CH₃ |
| A-93 | CH₂CH(CH₃)CH(CH₃)CH₂CH₂CH₂CH₂CH₂CH₃ |
| A-94 | CH₂CH₂CH₂C(CH₃)₂CH₂CH₂CH₂CH₂CH₃ |
| A-95 | CH(CH₃)CH₂CH(CH₃)CH₂CH₂CH₂CH₂CH₂CH₃ |
| A-96 | CH₂CH(CH₃)CH(CH₃)CH₂CH₂CH₂CH₂CH₂CH₃ |
| A-97 | CH(CH₃)CH₂CH₂CH(CH₃)CH₂CH₂CH₂CH₂CH₃ |
| A-98 | CH(CH₃)CH₂CH₂CH₂CH(CH₃)CH₂CH₂CH₂CH₃ |
| A-99 | CH(CH₃)CH₂CH₂CH₂CH₂CH(CH₃)CH₂CH₂CH₃ |
| A-100 | CH(CH₃)CH₂CH₂CH₂CH₂CH₂CH(CH₃)CH₂CH₃ |
| A-101 | CH(CH₃)CH₂CH₂CH₂CH₂CH₂CH₂CH(CH₃)₂ |
| A-102 | CH₂CH(CH₃)CH₂CH₂CH₂CH₂CH(CH₃)CH₂CH₃ |
| A-103 | CH₂CH(CH₃)CH₂CH₂CH₂CH₂CH(CH₃)CH₂CH₃ |
| A-104 | CH₂CH₂CH(CH₃)CH₂CH₂CH₂CH₂CH(CH₃)₂ |
| A-105 | CH₂CH(CH₃)CH₂CH₂CH₂CH₂C(CH₃)₃ |
| A-106 | CH₂CH₂CH₂CH₂CH₂CH₂CH₂CH₂CH₂CH₂CH₂CH₃ |
| A-107 | CH(CH₃)CH₂CH₂ CH₂CH₂CH₂CH₂CH₂CH₂CH₂CH₃ |
| A-108 | CH₂CH(CH₃)CH₂CH₂CH₂CH₂CH₂CH₂CH₂CH₂CH₃ |
| A-109 | CH₂CH₂CH(CH₃)CH₂CH₂CH₂CH₂CH₂CH₂CH₂CH₃ |
| A-110 | CH₂ CH₂CH₂CH(CH₃)CH₂CH₂CH₂CH₂CH₂CH₂CH₂ |
| A-111 | CH₂CH₂CH₂CH₂CH(CH₃)CH₂CH₂CH₂CH₂CH₂CH₃ |
| A-112 | CH₂CH₂CH₂CH₂CH₂CH(CH₃)CH₂CH₂CH₂CH₂CH₃ |
| A-113 | CH₂CH₂CH₂CH₂CH₂CH₂CH(CH₃)CH₂CH₂CH₂CH₃ |
| A-114 | CH₂CH₂CH₂CH₂CH₂CH₂CH₂CH(CH₃)CH₂CH₂CH₃ |
| A-115 | CH₂CH₂CH₂CH₂CH₂CH₂CH₂CH₂CH(CH₃)CH₂CH₃ |
| A-116 | CH₂CH₂CH₂CH₂CH₂CH₂CH₂CH₂CH₂CH(CH₃)₂ |
| A-117 | CH(CH₃)CH(CH₃)CH₂CH₂CH₂CH₂CH₂CH₂CH₂CH₃ |
| A-118 | CH₂CH(CH₃)CH(CH₃)CH₂CH₂CH₂CH₂CH₂CH₂CH₃ |
| A-119 | CH₂CH₂CH₂C(CH₃)₂CH₂CH₂CH₂CH₂CH₂CH₃ |
| A-120 | CH₂CH₂CH₂CH₂CH(CH₃)CH₂CH₂CH₂CH₂CH₂CH₃ |
| A-121 | CH(CH₃)CH₂CH(CH₃)CH₂CH₂CH₂CH₂ CH₂CH₂CH₃ |
| A-122 | CH(CH₃)CH₂CH₂CH(CH₃)CH₂CH₂CH₂ CH₂CH₂CH₃ |
| A-123 | CH(CH₃)CH₂CH₂CH₂CH(CH₃)CH₂CH₂CH₂CH₂CH₃ |
| A-124 | CH(CH₃)CH₂CH₂CH₂CH₂CH(CH₃)CH₂CH₂CH₂CH₃ |
| A-125 | CH(CH₃)CH₂CH₂CH₂CH₂CH₂CH(CH₃)CH₂CH₂CH₃ |
| A-126 | CH(CH₃)CH₂CH₂CH₂CH₂CH₂CH₂CH(CH₃)CH₂CH₃ |
| A-127 | CH₂CH(CH₃)CH₂CH₂CH₂CH₂CH(CH₃)CH₂CH₂CH₃ |
| A-128 | CH₂CH₂CH(CH₃)CH₂CH₂CH₂CH₂CH(CH₃)CH₂CH₃ |
| A-129 | CH₂CH₂CH₂CH(CH₃)CH₂CH₂CH₂CH(CH₃)CH₂CH₃ |
| A-130 | CH₂CH(CH₃)CH₂CH₂CH₂CH₂CH₂C(CH₃)₃ |
| A-131 | CH₂CH₂CH₂-O-CH₃ |
| A-132 | CH₂CH₂CH₂-O-CH₂CH₃ |
| A-133 | CH₂CH₂CH₂-O-CH₂CH₂CH₃ |
| A-134 | CH₂CH₂CH₂-O-CH₂CH₂CH₂CH₃ |
| A-135 | CH₂CH₂CH₂-O-CH₂CH₂CH₂CH₂CH₃ |
| A-136 | CH₂CH₂CH₂-O-CH₂CH₂CH₂CH₂CH₂CH₃ |
| A-137 | CH₂CH₂CH₂-O-CH₂CH₂CH₂CH₂CH₂CH₂CH₃ |
| A-138 | CH₂CH₂CH₂-O-CH₂CH₂CH₂CH₂CH₂CH₂CH₂CH₃ |
| A-139 | CH₂CH₂CH₂-O-CH₂CH₂CH₂CH₂CH₂CH₂CH₂CH₂CH₃ |
| A-140 | CH₂CH₂CH₂-O-CH(CH₃)₂ |
| A-141 | CH₂CH₂CH₂-O-C(CH₃)₃ |
| A-142 | CH₂CH₂CH₂-O-CH₂C(CH₃)₃ |
| A-143 | CH₂CH₂CH₂-O-CH(CH₃)CH₂C(CH₃)₃ |
| A-144 | CH₂CH₂CH₂-O-CH(CH₂CH₃)CH₂C(CH₃)₃ |
| A-145 | CH₂CH₂CH₂-O-CH₂CH(CH₃)CH₂CH(CH₃)₂ |
| A-146 | CH₂CH₂CH₂-O-CH₂CH(CH₂CH₃)CH₂CH₂CH₃ |
| A-147 | CH₂CH₂CH₂-O-CH₂CH₂CH(CH₃)CH₂CH(CH₃)₂ |
| A-148 | CH₂CH₂CH₂-O-CH₂CH₂CH(CH₃)CH₂C(CH₃)₃ |
| A-149 | CH₂CH₂CH₂-O-CH₂CH₂CH(CH₃)CH₂CH₂CH(CH₃)₂ |
| A-150 | CH₂CH₂CH₂-O-CH₂CH₂CH(CH₃)CH₂CH₂CH₂CH(CH₃)₂ |
| A-151 | CH₂CH₂CH₂CH₂-O-CH₃ |
| A-152 | CH₂CH₂CH₂CH₂-O-CH₂CH₃ |
| A-153 | CH₂CH₂CH₂CH₂-O-CH₂CH₂CH₃ |
| A-154 | CH₂CH₂CH₂CH₂-O-CH₂CH₂CH₂CH₃ |
| A-155 | CH₂CH₂CH₂CH₂-O-CH₂CH₂CH₂CH₂CH₃ |
| A-156 | CH₂CH₂CH₂CH₂-O-CH₂CH₂CH₂CH₂CH₂CH₃ |
| A-157 | CH₂CH₂CH₂CH₂-O-CH₂CH₂CH₂CH₂CH₂CH₂CH₃ |
| A-158 | CH₂CH₂CH₂CH₂-O-CH₂CH₂CH₂CH₂CH₂CH₂CH₂CH₃ |
| A-159 | CH₂CH₂CH₂CH₂-O-CH(CH₃)₂ |
| A-160 | CH₂CH₂CH₂CH₂-O-C(CH₃)₃ |
| A-161 | CH₂CH₂CH₂CH₂-O-CH₂C(CH₃)₃ |
| A-162 | CH₂CH₂CH₂CH₂-O-CH(CH₃)CH₂C(CH₃)₃ |
| A-163 | CH₂CH₂CH₂CH₂-O-CH(CH₂CH₃)CH₂C(CH₃)₃ |
| A-164 | CH₂CH₂CH₂CH₂-O-CH₂CH(CH₃)CH₂CH(CH₃)₂ |
| A-165 | CH₂CH₂CH₂CH₂-O-CH₂CH(CH₂CH₃)CH₂CH₂CH₃ |
| A-166 | CH₂CH₂CH₂CH₂-O-CH₂CH₂CH(CH₃)CH₂CH(CH₃)₂ |
| A-167 | CH₂CH₂CH₂CH₂-O-CH₂CH₂CH(CH₃)CH₂C(CH₃)₃ |
| A-168 | CH₂CH₂CH₂CH₂-O-CH₂CH₂CH(CH₃)CH₂CH₂CH(CH₃)₂ |
| A-169 | CH₂CH₂CH₂CH₂-O-CH₂CH₂CH(CH₃)CH₂CH₂CH₂CH(CH₃)₂ |
| A-170 | CH₂CH₂CH₂CH₂CH₂-O-CH₃ |
| A-171 | CH₂CH₂CH₂CH₂CH₂-O-CH₂CH₃ |
| A-172 | CH₂CH₂CH₂CH₂CH₂-O-CH₂CH₂CH₃ |
| A-173 | CH₂CH₂CH₂CH₂CH₂-O-CH₂CH₂CH₂CH₃ |
| A-174 | CH₂CH₂CH₂CH₂CH₂-O-CH₂CH₂CH₂CH₂CH₃ |
| A-175 | CH₂CH₂CH₂CH₂CH₂-O-CH₂CH₂CH₂CH₂CH₂CH₃ |
| A-176 | CH₂CH₂CH₂CH₂CH₂-O-CH₂CH₂CH₂CH₂CH₂CH₂CH₃ |
| A-177 | CH₂CH₂CH₂CH₂CH₂-O-CH₂CH₂CH₂CH₂CH₂CH₂CH₂CH₃ |
| A-178 | CH₂CH₂CH₂CH₂CH₂-O-CH(CH₃)₂ |
| A-179 | CH₂CH₂CH₂CH₂CH₂-O-C(CH₃)₃ |
| A-180 | CH₂CH₂CH₂CH₂CH₂-O-CH₂C(CH₃)₃ |
| A-181 | CH₂CH₂CH₂CH₂CH₂-O-CH(CH₃)CH₂C(CH₃)₃ |
| A-182 | CH₂CH₂CH₂CH₂CH₂-O-CH(CH₂CH₃)CH₂C(CH₃)₃ |
| A-183 | CH₂CH₂CH₂CH₂CH₂-O-CH₂CH(CH₃)CH₂CH(CH₃)₂ |
| A-184 | CH₂CH₂CH₂CH₂CH₂-O-CH₂CH(CH₂CH₃)CH₂CH₂CH₃ |
| A-185 | CH₂CH₂CH₂CH₂CH₂-O-CH₂CH₂CH(CH₃)CH₂CH₂CH(CH₃)₂ |
| A-186 | CH₂CH₂CH₂CH₂CH₂-O-CH₂CH₂CH(CH₃)CH₂CH(CH₃)₂ |
| A-187 | CH₂CH₂CH₂CH₂CH₂-O-CH₂CH₂CH(CH₃)CH₂C(CH₃)₃ |
| A-188 | Cyclopropyl |
| A-189 | Cyclopentyl |
| A-190 | Cyclohexyl |
| A-191 | CH=CH₂ |
| A-192 | CH₂CH=CH₂ |
| A-193 | CH=CHCH₃ |
| A-194 | C(CH₃)=CH₂ |
| A-195 | CH₂CH₂CH=CH₂ |
| A-196 | CH₂CH=CHCH₃ |
| A-197 | CH=CHCH₂CH₃ |
| A-198 | CH(CH₃)CH=CH₂ |
| A-199 | C(CH₃)=CHCH₃ |
| A-200 | CH=C(CH₃)₂ |
| A-201 | CH₂CH₂CH₂CH=CH₂ |
| A-202 | CH₂CH₂CH=CHCH₃ |
| A-203 | CH₂CH=CHCH₂CH₃ |
| A-204 | CH=CHCH₂CH₂CH₃ |
| A-205 | CH(CH₃)CH₂CH=CH₂ |
| A-206 | CH₂C(CH₃)=CHCH₃ |
| A-207 | CH₂CH=C(CH₃)₂ |
| A-208 | CH₂CH₂CH₂CH₂CH=CH₂ |
| A-209 | CH₂CH₂CH₂CH=CHCH₃ |
| A-210 | CH₂CH₂CH=CHCH₂CH₃ |
| A-211 | CH₂CH=CHCH₂CH₂CH₃ |
| A-212 | CH=CHCH₂CH₂CH₂CH₃ |
| A-213 | CH(CH₃)CH₂CH₂CH=CH₂ |
| A-214 | CH(CH₃)CH₂CH=CHCH₃ |
| A-215 | CH₂C(CH₃)=CHCH₂CH₃ |
| A-216 | CH₂CH₂CH=C(CH₃)₂ |
| A-217 | CH₂CH₂CH₂CH₂CH₂CH=CH₂ |
| A-218 | CH₂CH₂CH₂CH₂CH=CHCH₃ |
| A-219 | CH₂CH₂CH₂CH=CHCH₂CH₃ |
| A-220 | CH₂CH₂CH=CHCH₂CH₂CH₃ |
| A-221 | CH₂CH=CHCH₂CH₂CH₂CH₃ |
| A-222 | CH=CHCH₂CH₂CH₂CH₂CH₃ |
| A-223 | CH(CH₃)CH₂CH₂CH₂CH=CH₂ |
| A-224 | CH(CH₃)CH₂CH₂CH=CHCH₃ |
| A-225 | C(CH₃)=CHCH₂CH₂CH₂CH₃ |
| A-226 | CH₂CH₂CH₂CH=C(CH₃)₂ |
| A-227 | CH₂CH₂CH₂CH₂CH₂CH₂CH=CH₂ |
| A-228 | CH₂CH₂CH₂CH₂CH₂CH=CHCH₃ |
| A-229 | CH₂CH₂CH₂CH₂CH=CHCH₂CH₃ |
| A-230 | CH₂CH₂CH₂CH=CHCH₂CH₂CH₃ |
| A-231 | CH₂CH₂CH=CHCH₂CH₂CH₂CH₃ |
| A-232 | CH₂CH=CHCH₂CH₂CH₂CH₂CH₃ |
| A-233 | CH=CHCH₂CH₂CH₂CH₂CH₂CH₃ |
| A-234 | CH(CH₃)CH₂CH₂CH₂CH₂CH=CH₂ |
| A-235 | CH(CH₃)CH₂CH₂CH₂CH=CHCH₃ |
| A-236 | C(CH₃)=CHCH₂CH₂CH₂CH₂CH₃ |
| A-237 | CH₂CH₂CH₂CH₂CH=C(CH₃)₂ |
| A-238 | CH₂CH₂CH₂CH₂CH₂CH₂CH₂CH=CH₂ |
| A-239 | CH₂CH₂CH₂CH₂CH₂CH₂CH=CHCH₃ |
| A-240 | CH₂CH₂CH₂CH₂CH₂CH=CHCH₂CH₃ |
| A-241 | CH₂CH₂CH₂CH₂CH=CHCH₂CH₂CH₃ |
| A-242 | CH₂CH₂CH₂CH=CHCH₂CH₂CH₂CH₃ |
| A-243 | CH₂CH₂CH=CHCH₂CH₂CH₂CH₂CH₃ |
| A-244 | CH₂CH=CHCH₂CH₂CH₂CH₂CH₂CH₃ |
| A-245 | CH=CHCH₂CH₂CH₂CH₂CH₂CH₂CH₃ |
| A-246 | CH(CH₃)CH₂CH₂CH₂CH₂CH₂CH=CH₂ |
| A-247 | CH(CH₃)CH₂CH₂CH₂CH₂CH=CHCH₃ |
| A-248 | C(CH₃)=CHCH₂CH₂CH₂CH₂CH₂CH₃ |
| A-249 | CH₂CH₂CH₂CH₂CH₂CH=C(CH₃)₂ |
| A-250 | CH₂CH₂CH₂CH₂CH₂CH₂CH₂CH₂CH=CH₂ |
| A-251 | CH₂CH₂CH₂CH₂CH₂CH₂CH₂CH=CHCH₃ |
| A-252 | CH₂CH₂CH₂CH₂CH₂CH₂CH=CHCH₂CH₃ |
| A-253 | CH₂CH₂CH₂CH₂CH₂CH=CHCH₂CH₂CH₃ |
| A-254 | CH₂CH₂CH₂CH₂CH=CHCH₂CH₂CH₂CH₃ |
| A-255 | CH₂CH₂CH₂CH=CHCH₂CH₂CH₂CH₂CH₃ |
| A-256 | CH₂CH₂CH=CHCH₂CH₂CH₂CH₂CH₂CH₃ |
| A-257 | CH₂CH=CHCH₂CH₂CH₂CH₂CH₂CH₂CH₃ |
| A-258 | CH=CHCH₂CH₂CH₂CH₂CH₂CH₂CH₂CH₃ |
| A-259 | CH(CH₃)CH₂CH₂CH₂CH₂CH₂CH₂CH=CH₂ |
| A-260 | CH(CH₃)CH₂CH₂CH₂CH₂CH₂CH=CHCH₃ |
| A-261 | C(CH₃)=CHCH₂CH₂CH₂CH₂CH₂CH₂CH₃ |
| A-262 | CH₂CH₂CH₂CH₂CH₂CH₂CH=C(CH₃)₂ |
| A-263 | C=CH |
| A-264 | CH₂C≡CH |
| A-265 | C≡CCH₃ |
| A-266 | CH₂CH₂C≡CH |
| A-267 | CH₂C≡CCH₃ |
| A-268 | C≡CCH₂CH₃ |
| A-269 | CH(CH₃)C≡CH |
| A-270 | CH₂CH₂CH₂C≡CH |
| A-271 | CH₂CH₂C≡CCH₃ |
| A-272 | CH₂C≡CCH₂CH₃ |
| A-273 | C≡CCH₂CH₂CH₃ |
| A-274 | CH(CH₃)CH₂C≡CH |
| A-275 | CH₂CH₂CH₂CH₂C≡CH |
| A-276 | CH₂CH₂CH₂C≡CCH₃ |
| A-277 | CH₂CH₂C≡CCH₂CH₃ |
| A-278 | CH₂C≡CCH₂CH₂CH₃ |
| A-279 | C≡CCH₂CH₂CH₂CH₃ |
| A-280 | CH(CH₃)CH₂CH₂C≡CH |
| A-281 | CH(CH₃)CH₂C≡CCH₃ |
| A-282 | CH₂CH₂CH₂CH₂CH₂C≡CH |
| A-283 | CH₂CH₂CH₂CH₂C≡CCH₃ |
| A-284 | CH₂CH₂CH₂C≡CCH₂CH₃ |
| A-285 | CH₂CH₂C≡CCH₂CH₂CH₃ |
| A-286 | CH₂C≡CCH₂CH₂CH₂CH₃ |
| A-287 | C≡CCH₂CH₂CH₂CH₂CH₃ |
| A-288 | CH(CH₃)CH₂CH₂CH₂C≡CH |
| A-289 | CH(CH₃)CH₂CH₂C≡CCH₃ |
| A-290 | CH(CH₃)CH₂C≡CCH₂CH₃ |
| A-291 | CH₂CH₂CH₂CH₂CH₂CH₂C≡CH |
| A-292 | CH₂CH₂CH₂CH₂CH₂C≡CCH₃ |
| A-293 | CH₂CH₂CH₂CH₂C≡CCH₂CH₃ |
| A-294 | CH₂CH₂CH₂C≡CCH₂CH₂CH₃ |
| A-295 | CH₂CH₂C≡CCH₂CH₂CH₂CH₃ |
| A-296 | CH₂C≡CCH₂CH₂CH₂CH₂CH₃ |
| A-297 | C≡CCH₂CH₂CH₂CH₂CH₂CH₃ |
| A-298 | CH(CH₃)CH₂CH₂CH₂CH₂C≡CH |
| A-299 | CH(CH₃)CH₂CH₂CH₂C≡CCH₃ |
| A-300 | CH₂CH₂CH₂CH₂CH₂CH₂CH₂C≡CH |
| A-301 | CH₂CH₂CH₂CH₂CH₂CH₂C≡CCH₃ |
| A-302 | CH₂CH₂CH₂CH₂CH₂C≡CCH₂CH₃ |
| A-303 | CH₂CH₂CH₂CH₂C≡CCH₂CH₂CH₃ |
| A-304 | CH₂CH₂CH₂C≡CCH₂CH₂CH₂CH₃ |
| A-305 | CH₂CH₂C≡CCH₂CH₂CH₂CH₂CH₃ |
| A-306 | CH₂C≡CCH₂CH₂CH₂CH₂CH₂CH₃ |
| A-307 | C≡CCH₂CH₂CH₂CH₂CH₂CH₂CH₃ |
| A-308 | CH(CH₃)CH₂CH₂CH₂CH₂CH₂C≡CH |
| A-309 | CH(CH₃)CH₂CH₂CH₂CH₂C≡CCH₃ |
| A-310 | CH₂CH₂CH₂CH₂CH₂CH₂CH₂CH₂C≡CH |
| A-311 | CH₂CH₂CH₂CH₂CH₂CH₂CH₂C≡CCH₃ |
| A-312 | CH₂CH₂CH₂CH₂CH₂CH₂C≡CCH₂CH₃ |
| A-313 | CH₂CH₂CH₂CH₂CH₂C≡CCH₂CH₂CH₃ |
| A-314 | CH₂CH₂CH₂CH₂C≡CCH₂CH₂CH₂CH₃ |
| A-315 | CH₂CH₂CH₂C≡CCH₂CH₂CH₂CH₂CH₃ |
| A-316 | CH₂CH₂C≡CCH₂CH₂CH₂CH₂CH₂CH₃ |
| A-317 | CH₂C≡CCH₂CH₂CH₂CH₂CH₂CH₂CH₃ |
| A-318 | C≡CCH₂CH₂CH₂CH₂CH₂CH₂CH₂CH₃ |
| A-319 | CH(CH₃)CH₂CH₂CH₂CH₂CH₂CH₂C≡CH |
| A-320 | CH(CH₃)CH₂CH₂CH₂CH₂CH₂C≡CCH₃ |
| A-321 | CH₂CH₂CN |
| A-322 | CH₂CH₂CH₂CN |
| A-323 | CH₂CH₂CH₂CH₂CN |
| A-324 | CH₂CH₂CH₂CH₂CH₂CN |
| A-325 | CH₂CH₂CH₂CH₂CH₂CH₂CN |
| A-326 | CH₂CH₂CH₂CH₂CH₂CH₂CH₂CN |
| A-327 | CH₂CH₂CH₂CH₂CH₂CH₂CH₂CH₂CN |
| A-328 | CH₂CH₂CH₂CH₂CH₂CH₂CH₂CH₂CH₂CN |
| A-329 | CH₂CH₂CH₂CH₂CH₂CH₂CH₂CH₂CH₂CH₂CN |

### Tabelle 46

Verbindungen der Formel I.1, in denen R¹ für eine direkt gebundene Gruppe G steht, deren Substituenten für eine Verbindung jeweils einer Zeile der Tabelle B entspricht und R² Methyl und R³ Wasserstoff bedeutet

### Tabelle 47

Verbindungen der Formel I.1, in denen R¹ für eine direkt gebundene Gruppe G steht, deren Substituenten für eine Verbindung jeweils einer Zeile der Tabelle B entspricht und R² Ethyl und R³ Wasserstoff bedeutet

### Tabelle 48

Verbindungen der Formel I.1, in denen R¹ für eine direkt gebundene Gruppe G steht, deren Substituenten für eine Verbindung jeweils einer Zeile der Tabelle B entspricht und R² n-Propyl und R³ Wasserstoff bedeutet

### Tabelle 49

Verbindungen der Formel I.1, in denen R¹ für eine direkt gebundene Gruppe G steht, deren Substituenten für eine Verbindung jeweils einer Zeile der Tabelle B entspricht und R² Methoxymethyl und R³ Wasserstoff bedeutet

### Tabelle 50

Verbindungen der Formel I.1, in denen R¹ für eine direkt gebundene Gruppe G steht, deren Substituenten für eine Verbindung jeweils einer Zeile der Tabelle B entspricht und R² Ethoxymethyl und R³ Wasserstoff bedeutet.

### Tabelle 51

Verbindungen der Formel I.1, in denen R¹ für eine direkt gebundene Gruppe G steht, deren Substituenten für eine Verbindung jeweils einer Zeile der Tabelle B entspricht und R² n-Propoxymethyl und R³ Wasserstoff bedeutet.

### Tabelle 52

Verbindungen der Formel I.1, in denen R¹ für eine direkt gebundene Gruppe G steht, deren Substituenten für eine Verbindung jeweils einer Zeile der Tabelle B entspricht und R² Methoxyethyl und R³ Wasserstoff bedeutet.

### Tabelle 53

Verbindungen der Formel I.1, in denen R¹ für eine direkt gebundene Gruppe G steht, deren Substituenten für eine Verbindung jeweils einer Zeile der Tabelle B entspricht und R² Ethoxyethyl und R³ Wasserstoff bedeutet.

### Tabelle 54

Verbindungen der Formel I.1, in denen R¹ für eine direkt gebundene Gruppe G steht, deren Substituenten für eine Verbindung jeweils einer Zeile der Tabelle B entspricht und R² n-Propoxyethyl und R³ Wasserstoff bedeutet.

### Tabelle 55

Verbindungen der Formel I.1, in denen R¹ für eine direkt gebundene Gruppe G steht, deren Substituenten für eine Verbindung jeweils einer Zeile der Tabelle B entspricht und R² 3-Methoxy-n-propyl und R³ Wasserstoff bedeutet.

### Tabelle 56

Verbindungen der Formel I.1, in denen R¹ für eine direkt gebundene Gruppe G steht, deren Substituenten für eine Verbindung jeweils einer Zeile der Tabelle B entspricht und R² 3-Ethoxy-n-propyl und R³ Wasserstoff bedeutet.

### Tabelle 57

Verbindungen der Formel I.1, in denen R¹ für eine direkt gebundene Gruppe G steht, deren Substituenten für eine Verbindung jeweils einer Zeile der Tabelle B entspricht und R² 3-n-Propoxy-n-propyl und R³ Wasserstoff bedeutet.

### Tabelle 58

Verbindungen der Formel I.1, in denen R¹ für eine direkt gebundene Gruppe G steht, deren Substituenten für eine Verbindung jeweils einer Zeile der Tabelle B entspricht und R² Ethenyl und R³ Wasserstoff bedeutet

### Tabelle 59

Verbindungen der Formel I.1, in denen R¹ für eine direkt gebundene Gruppe G steht, deren Substituenten für eine Verbindung jeweils einer Zeile der Tabelle B entspricht und R² Allyl und R³ Wasserstoff bedeutet

### Tabelle 60

Verbindungen der Formel I.1, in denen R¹ für eine direkt gebundene Gruppe G steht, deren Substituenten für eine Verbindung jeweils einer Zeile der Tabelle B entspricht und R² Trifluormethyl und R³ Wasserstoff bedeutet.

### Tabelle 61

Verbindungen der Formel I.1, in denen R¹ für eine über Methylen gebundene Gruppe G steht, deren Substituenten für eine Verbindung jeweils einer Zeile der Tabelle B entspricht und R² Methyl und R³ Wasserstoff bedeutet

### Tabelle 62

Verbindungen der Formel I.1, in denen R¹ für eine über Methylen gebundene Gruppe G steht, deren Substituenten für eine Verbindung jeweils einer Zeile der Tabelle B entspricht und R² Ethyl und R³ Wasserstoff bedeutet

### Tabelle 63

Verbindungen der Formel I.1, in denen R¹ für eine über Methylen gebundene Gruppe G steht, deren Substituenten für eine Verbindung jeweils einer Zeile der Tabelle B entspricht und R² n-Propyl und R³ Wasserstoff bedeutet

### Tabelle 64

Verbindungen der Formel I.1, in denen R¹ für eine über Methylen gebundene Gruppe G steht, deren Substituenten für eine Verbindung jeweils einer Zeile der Tabelle B entspricht und R² Methoxymethyl und R³ Wasserstoff bedeutet

### Tabelle 65

Verbindungen der Formel I.1, in denen R¹ für eine über Methylen gebundene Gruppe G steht, deren Substituenten für eine Verbindung jeweils einer Zeile der Tabelle B entspricht und R² Ethoxymethyl und R³ Wasserstoff bedeutet.

### Tabelle 66

Verbindungen der Formel I.1, in denen R¹ für eine über Methylen gebundene Gruppe G steht, deren Substituenten für eine Verbindung jeweils einer Zeile,der Tabelle B entspricht und R² n-Propoxymethyl und R³ Wasserstoff bedeutet.

### Tabelle 67

Verbindungen der Formel I.1, in denen R¹ für eine über Methylen gebundene Gruppe G steht, deren Substituenten für eine Verbindung jeweils einer Zeile der Tabelle B entspricht und R² Methoxyethyl und R³ Wasserstoff bedeutet.

### Tabelle 68

Verbindungen der Formel I.1, in denen R¹ für eine über Methylen gebundene Gruppe G steht, deren Substituenten für eine Verbindung jeweils einer Zeile der Tabelle B entspricht und R² Ethoxyethyl und R³ Wasserstoff bedeutet.

### Tabelle 69

Verbindungen der Formel I.1, in denen R¹ für eine über Methylen gebundene Gruppe G steht, deren Substituenten für eine Verbindung jeweils einer Zeile der Tabelle B entspricht und R² n-Propoxyethyl und R³ Wasserstoff bedeutet.

### Tabelle 70

Verbindungen der Formel I.1, in denen R¹ für eine über Methylen gebundene Gruppe G steht, deren Substituenten für eine Verbindung jeweils einer Zeile der Tabelle B entspricht und R² 3-Methoxy-n-propyl und R³ Wasserstoff bedeutet.

### Tabelle 71

Verbindungen der Formel I.1, in denen R¹ für eine über Methylen gebundene Gruppe G steht, deren Substituenten für eine Verbindung jeweils einer Zeile der Tabelle B entspricht und R² 3-Ethoxy-n-propyl und R³ Wasserstoff bedeutet.

### Tabelle 72

Verbindungen der Formel I.1, in denen R¹ für eine über Methylen gebundene Gruppe G steht, deren Substituenten für eine Verbindung jeweils einer Zeile der Tabelle B entspricht und R² 3-n-Propoxy-n-propyl und R³ Wasserstoff bedeutet.

### Tabelle 73

Verbindungen der Formel I.1, in denen R¹ für eine über Methylen gebundene Gruppe G steht, deren Substituenten für eine Verbindung jeweils einer Zeile der Tabelle B entspricht und R² Ethenyl und R³ Wasserstoff bedeutet

### Tabelle 74

Verbindungen der Formel I.1, in denen R¹ für eine über Methylen gebundene Gruppe G steht, deren Substituenten für eine Verbindung jeweils einer Zeile der Tabelle B entspricht und R² Allyl und R³ Wasserstoff bedeutet

### Tabelle 75

Verbindungen der Formel I.1, in denen R¹ für eine über Methylen gebundene Gruppe G steht, deren Substituenten für eine Verbindung jeweils einer Zeile der Tabelle B entspricht und R² Trifluormethyl und R³ Wasserstoff bedeutet.

### Tabelle 76

Verbindungen der Formel I.2, in denen R¹ für eine direkt gebundene Gruppe G steht, deren Substituenten für eine Verbindung jeweils einer Zeile der Tabelle B entspricht und R² Methyl und R³ Wasserstoff bedeutet

### Tabelle 77

Verbindungen der Formel I.2, in denen R¹ für eine direkt gebundene Gruppe G steht, deren Substituenten für eine Verbindung jeweils einer Zeile der Tabelle B entspricht und R² Ethyl und R³ Wasserstoff bedeutet

### Tabelle 78

Verbindungen der Formel I.2, in denen R¹ für eine direkt gebundene Gruppe G steht, deren Substituenten für eine Verbindung jeweils einer Zeile der Tabelle B entspricht und R² n-Propyl und R³ Wasserstoff bedeutet

### Tabelle 79

Verbindungen der Formel I.2, in denen R¹ für eine direkt gebundene Gruppe G steht, deren Substituenten für eine Verbindung jeweils einer Zeile der Tabelle B entspricht und R² Methoxymethyl und R³ Wasserstoff bedeutet

### Tabelle 80

Verbindungen der Formel I.2, in denen R¹ für eine direkt gebundene Gruppe G steht, deren Substituenten für eine Verbindung jeweils einer Zeile der Tabelle B entspricht und R² Ethoxymethyl und R³ Wasserstoff bedeutet.

### Tabelle 81

Verbindungen der Formel I.2, in denen R¹ für eine direkt gebundene Gruppe G steht, deren Substituenten für eine Verbindung jeweils einer Zeile der Tabelle B entspricht und R² n-Propoxymethyl und R³ Wasserstoff bedeutet.

### Tabelle 82

Verbindungen der Formel I.2, in denen R¹ für eine direkt gebundene Gruppe G steht, deren Substituenten für eine Verbindung jeweils einer Zeile der Tabelle B entspricht und R² Methoxyethyl und R³ Wasserstoff bedeutet.

### Tabelle 83

Verbindungen der Formel I.2, in denen R¹ für eine direkt gebundene Gruppe G steht, deren Substituenten für eine Verbindung jeweils einer Zeile der Tabelle B entspricht und R² Ethoxyethyl und R³ Wasserstoff bedeutet.

### Tabelle 84

Verbindungen der Formel I.2, in denen R¹ für eine direkt gebundene Gruppe G steht, deren Substituenten für eine Verbindung jeweils einer Zeile der Tabelle B entspricht und R² n-Propoxyethyl und R³ Wasserstoff bedeutet.

### Tabelle 85

Verbindungen der Formel I.2, in denen R¹ für eine direkt gebundene Gruppe G steht, deren Substituenten für eine Verbindung jeweils einer Zeile der Tabelle B entspricht und R² 3-Methoxy-n-propyl und R³ Wasserstoff bedeutet.

### Tabelle 86

Verbindungen der Formel I.2, in denen R¹ für eine direkt gebundene Gruppe G steht, deren Substituenten für eine Verbindung jeweils einer Zeile der Tabelle B entspricht und R² 3-Ethoxy-n-propyl und R³ Wasserstoff bedeutet.

### Tabelle 87

Verbindungen der Formel I.2, in denen R¹ für eine direkt gebundene Gruppe G steht, deren Substituenten für eine Verbindung jeweils einer Zeile der Tabelle B entspricht und R² 3-n-Propoxy-n-propyl und R³ Wasserstoff bedeutet.

### Tabelle 88

Verbindungen der Formel I.2, in denen R¹ für eine direkt gebundene Gruppe G steht, deren Substituenten für eine Verbindung jeweils einer Zeile der Tabelle B entspricht und R² Ethenyl und R³ Wasserstoff bedeutet

### Tabelle 89

Verbindungen der Formel I.2, in denen R¹ für eine direkt gebundene Gruppe G steht, deren Substituenten für eine Verbindung jeweils einer Zeile der Tabelle B entspricht und R² Allyl und R³ Wasserstoff bedeutet

### Tabelle 90

Verbindungen der Formel I.2, in denen R¹ für eine direkt gebundene Gruppe G steht, deren Substituenten für eine Verbindung jeweils einer Zeile der Tabelle B entspricht und R² Trifluormethyl und R³ Wasserstoff bedeutet.

### Tabelle 91

Verbindungen der Formel I.2, in denen R¹ für eine über Methylen gebundene Gruppe G steht, deren Substituenten für eine Verbindung jeweils einer Zeile der Tabelle B entspricht und R² Methyl und R³ Wasserstoff bedeutet

### Tabelle 92

Verbindungen der Formel I.2, in denen R¹ für eine über Methylen gebundene Gruppe G steht, deren Substituenten für eine Verbindung jeweils einer Zeile der Tabelle B entspricht und R² Ethyl und R³ Wasserstoff bedeutet

### Tabelle 93

Verbindungen der Formel I.2, in denen R¹ für eine über Methylen gebundene Gruppe G steht, deren Substituenten für eine Verbindung jeweils einer Zeile der Tabelle B entspricht und R² n-Propyl und R³ Wasserstoff bedeutet

### Tabelle 94

Verbindungen der Formel I.2, in denen R¹ für eine über Methylen gebundene Gruppe G steht, deren Substituenten für eine Verbindung jeweils einer Zeile der Tabelle B entspricht und R² Methoxymethyl und R³ Wasserstoff bedeutet

### Tabelle 95

Verbindungen der Formel I.2, in denen R¹ für eine über Methylen gebundene Gruppe G steht, deren Substituenten für eine Verbindung jeweils einer Zeile der Tabelle B entspricht und R² Ethoxymethyl und R³ Wasserstoff bedeutet.

### Tabelle 96

Verbindungen der Formel I.2, in denen R¹ für eine über Methylen gebundene Gruppe G steht, deren Substituenten für eine Verbindung jeweils einer Zeile der Tabelle B entspricht und R² n-Propoxymethyl und R³ Wasserstoff bedeutet.

### Tabelle 97

Verbindungen der Formel I.2, in denen R¹ für eine über Methylen gebundene Gruppe G steht, deren Substituenten für eine Verbindung jeweils einer Zeile der Tabelle B entspricht und R² Methoxyethyl und R³ Wasserstoff bedeutet.

### Tabelle 98

Verbindungen der Formel I.2, in denen R¹ für eine über Methylen gebundene Gruppe G steht, deren Substituenten für eine Verbindung jeweils einer Zeile der Tabelle B entspricht und R² Ethoxyethyl und R³ Wasserstoff bedeutet.

### Tabelle 99

Verbindungen der Formel I.2, in denen R¹ für eine über Methylen gebundene Gruppe G steht, deren Substituenten für eine Verbindung jeweils einer Zeile der Tabelle B entspricht und R² n-Propoxyethyl und R³ Wasserstoff bedeutet.

### Tabelle 100

Verbindungen der Formel I.2, in denen R¹ für eine über Methylen gebundene Gruppe G steht, deren Substituenten für eine Verbindung jeweils einer Zeile der Tabelle B entspricht und R² 3-Methoxy-n-propyl und R³ Wasserstoff bedeutet.

### Tabelle 101

Verbindungen der Formel I.2, in denen R¹ für eine über Methylen gebundene Gruppe G steht, deren Substituenten für eine Verbindung jeweils einer Zeile der Tabelle B entspricht und R² 3-Ethoxy-n-propyl und R³ Wasserstoff bedeutet.

### Tabelle 102

Verbindungen der Formel I.2, in denen R¹ für eine über Methylen gebundene Gruppe G steht, deren Substituenten für eine Verbindung jeweils einer Zeile der Tabelle B entspricht und R² 3-n-Propoxy-n-propyl und R³ Wasserstoff bedeutet.

### Tabelle 103

Verbindungen der Formel I.2, in denen R¹ für eine über Methylen gebundene Gruppe G steht, deren Substituenten für eine Verbindung jeweils einer Zeile der Tabelle B entspricht und R² Ethenyl und R³ Wasserstoff bedeutet

### Tabelle 104

Verbindungen der Formel I.2, in denen R¹ für eine über Methylen gebundene Gruppe G steht, deren Substituenten für eine Verbindung jeweils einer Zeile der Tabelle B entspricht und R² Allyl und R³ Wasserstoff bedeutet

### Tabelle 105

Verbindungen der Formel I.2, in denen R¹ für eine über Methylen gebundene Gruppe G steht, deren Substituenten für eine Verbindung jeweils einer Zeile der Tabelle B entspricht und R² Trifluormethyl und R³ Wasserstoff bedeutet.

### Tabelle 106

Verbindungen der Formel I.3, in denen R¹ für eine direkt gebundene Gruppe G steht, deren Substituenten für eine Verbindung jeweils einer Zeile der Tabelle B entspricht und R² Methyl und R³ Wasserstoff bedeutet

### Tabelle 107

Verbindungen der Formel I.3, in denen R¹ für eine direkt gebundene Gruppe G steht, deren Substituenten für eine Verbindung jeweils einer Zeile der Tabelle B entspricht und R² Ethyl und R³ Wasserstoff bedeutet

### Tabelle 108

Verbindungen der Formel I.3, in denen R¹ für eine direkt gebundene Gruppe G steht, deren Substituenten für eine Verbindung jeweils einer Zeile der Tabelle B entspricht und R² n-Propyl und R³ Wasserstoff bedeutet

### Tabelle 109

Verbindungen der Formel I.3, in denen R¹ für eine direkt gebundene Gruppe G steht, deren Substituenten für eine Verbindung jeweils einer Zeile der Tabelle B entspricht und R² Methoxymethyl und R³ Wasserstoff bedeutet

### Tabelle 110

Verbindungen der Formel I.3, in denen R¹ für eine direkt gebundene Gruppe G steht, deren Substituenten für eine Verbindung jeweils einer Zeile der Tabelle B entspricht und R² Ethoxymethyl und R³ Wasserstoff bedeutet.

### Tabelle 111

Verbindungen der Formel I.3, in denen R¹ für eine direkt gebundene Gruppe G steht, deren Substituenten für eine Verbindung jeweils einer Zeile der Tabelle B entspricht und R² n-Propoxymethyl und R³ Wasserstoff bedeutet.

### Tabelle 112

Verbindungen der Formel I.3, in denen R¹ für eine direkt gebundene Gruppe G steht, deren Substituenten für eine Verbindung jeweils einer Zeile der Tabelle B entspricht und R² Methoxyethyl und R³ Wasserstoff bedeutet.

### Tabelle 113

Verbindungen der Formel I.3, in denen R¹ für eine direkt gebundene Gruppe G steht, deren Substituenten für eine Verbindung jeweils einer Zeile der Tabelle B entspricht und R² Ethoxyethyl und R³ Wasserstoff bedeutet.

### Tabelle 114

Verbindungen der Formel I.3, in denen R¹ für eine direkt gebundene Gruppe G steht, deren Substituenten für eine Verbindung jeweils einer Zeile der Tabelle B entspricht und R² n-Propoxyethyl und R³ Wasserstoff bedeutet.

### Tabelle 115

Verbindungen der Formel I.3, in denen R¹ für eine direkt gebundene Gruppe G steht, deren Substituenten für eine Verbindung jeweils einer Zeile der Tabelle B entspricht und R² 3-Methoxy-n-propyl und R³ Wasserstoff bedeutet.

### Tabelle 116

Verbindungen der Formel I.3, in denen R¹ für eine direkt gebundene Gruppe G steht, deren Substituenten für eine Verbindung jeweils einer Zeile der Tabelle B entspricht und R² 3-Ethoxy-n-propyl und R³ Wasserstoff bedeutet.

### Tabelle 117

Verbindungen der Formel I.3, in denen R¹ für eine direkt gebundene Gruppe G steht, deren Substituenten für eine Verbindung jeweils einer Zeile der Tabelle B entspricht und R² 3-n-Propoxy-n-propyl und R³ Wasserstoff bedeutet.

### Tabelle 118

Verbindungen der Formel I.3, in denen R¹ für eine direkt gebundene Gruppe G steht, deren Substituenten für eine Verbindung jeweils einer Zeile der Tabelle B entspricht und R² Ethenyl und R³ Wasserstoff bedeutet

### Tabelle 119

Verbindungen der Formel I.3, in denen R¹ für eine direkt gebundene Gruppe G steht, deren Substituenten für eine Verbindung jeweils einer Zeile der Tabelle B entspricht und R² Allyl und R³ Wasserstoff bedeutet

### Tabelle 120

Verbindungen der Formel I.3, in denen R¹ für eine direkt gebundene Gruppe G steht, deren Substituenten für eine Verbindung jeweils einer Zeile der Tabelle B entspricht und R² Trifluormethyl und R³ Wasserstoff bedeutet.

### Tabelle 121

Verbindungen der Formel I.3, in denen R¹ für eine über Methylen gebundene Gruppe G steht, deren Substituenten für eine Verbindung jeweils einer Zeile der Tabelle B entspricht und R² Methyl und R³ Wasserstoff bedeutet

### Tabelle 122

Verbindungen der Formel I.3, in denen R¹ für eine über Methylen gebundene Gruppe G steht, deren Substituenten für eine Verbindung jeweils einer Zeile der Tabelle B entspricht und R² Ethyl und R³ Wasserstoff bedeutet

### Tabelle 123

Verbindungen der Formel I.3, in denen R¹ für eine über Methylen gebundene Gruppe G steht, deren Substituenten für eine Verbindung jeweils einer Zeile der Tabelle B entspricht und R² n-Propyl und R³ Wasserstoff bedeutet

### Tabelle 124

Verbindungen der Formel I.3, in denen R¹ für eine über Methylen gebundene Gruppe G steht, deren Substituenten für eine Verbindung jeweils einer Zeile der Tabelle B entspricht und R² Methoxymethyl und R³ Wasserstoff bedeutet

### Tabelle 125

Verbindungen der Formel I.3, in denen R¹ für eine über Methylen gebundene Gruppe G steht, deren Substituenten für eine Verbindung jeweils einer Zeile der Tabelle B entspricht und R² Ethoxymethyl und R³ Wasserstoff bedeutet.

### Tabelle 126

Verbindungen der Formel I.3, in denen R¹ für eine über Methylen gebundene Gruppe G steht, deren Substituenten für eine Verbindung jeweils einer Zeile der Tabelle B entspricht und R² n-Propoxymethyl und R³ Wasserstoff bedeutet.

### Tabelle 127

Verbindungen der Formel I.3, in denen R¹ für eine über Methylen gebundene Gruppe G steht, deren Substituenten für eine Verbindung jeweils einer Zeile der Tabelle B entspricht und R² Methoxyethyl und R³ Wasserstoff bedeutet.

### Tabelle 128

Verbindungen der Formel I.3, in denen R¹ für eine über Methylen gebundene Gruppe G steht, deren Substituenten für eine Verbindung jeweils einer Zeile der Tabelle B entspricht und R² Ethoxyethyl und R³ Wasserstoff bedeutet.

### Tabelle 129

Verbindungen der Formel I.3, in denen R¹ für eine über Methylen gebundene Gruppe G steht, deren Substituenten für eine Verbindung jeweils einer Zeile der Tabelle B entspricht und R² n-Propoxyethyl und R³ Wasserstoff bedeutet.

### Tabelle 130

Verbindungen der Formel I.3, in denen R¹ für eine über Methylen gebundene Gruppe G steht, deren Substituenten für eine Verbindung jeweils einer Zeile der Tabelle B entspricht und R² 3-Methoxy-n-propyl und R³ Wasserstoff bedeutet.

### Tabelle 131

Verbindungen der Formel I.3, in denen R¹ für eine über Methylen gebundene Gruppe G steht, deren Substituenten für eine Verbindung jeweils einer Zeile der Tabelle B entspricht und R² 3-Ethoxy-n-propyl und R³ Wasserstoff bedeutet.

### Tabelle 132

Verbindungen der Formel I.3, in denen R¹ für eine über Methylen gebundene Gruppe G steht, deren Substituenten für eine Verbindung jeweils einer Zeile der Tabelle B entspricht und R² 3-n-Propoxy-n-propyl und R³ Wasserstoff bedeutet.

### Tabelle 133

Verbindungen der Formel I.3, in denen R¹ für eine über Methylen gebundene Gruppe G steht, deren Substituenten für eine Verbindung jeweils einer Zeile der Tabelle B entspricht und R² Ethenyl und R³ Wasserstoff bedeutet

### Tabelle 134

Verbindungen der Formel I.3, in denen R¹ für eine über Methylen gebundene Gruppe G steht, deren Substituenten für eine Verbindung jeweils einer Zeile der Tabelle B entspricht und R² Allyl und R³ Wasserstoff bedeutet

### Tabelle 135

Verbindungen der Formel I.3, in denen R¹ für eine über Methylen gebundene Gruppe G steht, deren Substituenten für eine Verbindung jeweils einer Zeile der Tabelle B entspricht und R² Trifluormethyl und R³ Wasserstoff bedeutet.

**Tabelle B**

| **Nr.** | **L¹** | **L²** | **L³** | **L⁴** | **L⁵** |
|---|---|---|---|---|---|
| B-1 | CH₃ | H | H | H | H |
| B-2 | H | CH₃ | H | H | H |
| B-3 | H | H | CH₃ | H | H |
| B-4 | CH₃ | H | CH₃ | H | H |
| B-5 | H | CH₃ | CH₃ | H | H |
| B-6 | CH₂CH₃ | H | H | H | H |
| B-7 | H | CH₂CH₃ | H | H | H |
| B-8 | H | H | CH₂CH₃ | H | H |
| B-9 | CH₂CH₃ | H | CH₂CH₃ | H | H |
| B-10 | H | CH₂CH₃ | CH₂CH₃ | H | H |
| B-11 | CH₂CH₂CH₃ | H | H | H | H |
| B-12 | H | CH₂CH₂CH₃ | H | H | H |
| B-13 | H | H | CH₂CH₂CH₃ | H | H |
| B-14 | CH₂CH₂CH₃ | H | CH₂CH₂CH₃ | H | H |
| B-15 | H | CH₂CH₂CH₃ | CH₂CH₂CH₃ | H | H |
| B-16 | CH(CH₃)₂ | H | H | H | H |
| B-17 | H | CH(CH₃)₂ | H | H | H |
| B-18 | H | H | CH(CH₃)₂ | H | H |
| B-19 | CH(CH₃)₂ | H | CH(CH₃)₂ | H | H |
| B-20 | H | CH(CH₃)₂ | CH(CH₃)₂ | H | H |
| B-21 | CH₂CH₂CH₂CH₃ | H | H | H | H |
| B-22 | H | CH₂CH₂CH₂CH₃ | H | H | H |
| B-23 | H | H | CH₂CH₂CH₂CH₃ | H | H |
| B-24 | CH₂CH₂CH₂CH₃ | H | CH₂CH₂CH₂CH₃ | H | H |
| B-25 | H | CH₂CH₂CH₂CH₃ | CH₂CH₂CH₂CH₃ | H | H |
| B-26 | CH(CH₃)CH₂CH₃ | H | H | H | H |
| B-27 | H | CH(CH₃)CH₂CH₃ | H | H | H |
| B-28 | H | H | CH(CH₃)CH₂CH₃ | H | H |
| B-29 | CH(CH₃)CH₂CH₃ | H | CH(CH₃)CH₂CH₃ | H | H |
| B-30 | H | CH(CH₃)CH₂CH₃ | CH(CH₃)CH₂CH₃ | H | H |
| B-31 | C(CH₃)₃ | H | H | H | H |
| B-32 | H | C(CH₃)₃ | H | H | H |
| B-33 | H | H | C(CH₃)₃ | H | H |
| B-34 | C(CH₃)₃ | H | C(CH₃)₃ | H | H |
| B-35 | H | C(CH₃)₃ | C(CH₃)₃ | H | H |
| B-36 | CH₂CH(CH₃)CH₂CH₃ | H | H | H | H |
| B-37 | H | CH₂CH(CH₃)CH₂CH₃ | H | H | H |
| B-38 | H | H | CH₂CH(CH₃)CH₂CH₃ | H | H |
| B-39 | CH₃ | H | CH₂CH(CH₃)CH₂CH₃ | H | H |
| B-40 | H | CH₂CH(CH₃)CH₂CH₃ | CH₂CH(CH₃)CH₂CH₃ | H | H |
| B-41 | CH₂C(CH₃)₃ | H | H | H | H |
| B-42 | H | CH₂C(CH₃)₃ | H | H | H |
| B-43 | H | H | CH₂C(CH₃)₃ | H | H |
| B-44 | CH₂C(CH₃)₃ | H | CH₂C(CH₃)₃ | H | H |
| B-45 | H | CH₂C(CH₃)₃ | CH₂C(CH₃)₃ | H | H |
| B-46 | Cl | H | H | H | H |
| B-47 | H | Cl | H | H | H |
| B-48 | H | H | Cl | H | H |
| B-49 | Cl | H | Cl | H | H |
| B-50 | H | Cl | Cl | H | H |
| B-51 | F | H | H | H | H |
| B-52 | H | F | H | H | H |
| B-53 | H | H | F | H | H |
| B-54 | F | H | F | H | H |
| B-55 | H | F | F | H | H |
| B-56 | Br | H | H | H | H |
| B-57 | H | Br | H | H | H |
| B-58 | H | H | Br | H | H |
| B-59 | Br | H | Br | H | H |
| B-60 | H | Br | Br | H | H |
| B-61 | CF₃ | H | H | H | H |
| B-62 | H | CF₃ | H | H | H |
| B-63 | H | H | CF₃ | H | H |
| B-64 | CF₃ | H | CF₃ | H | H |
| B-65 | H | CF₃ | CF₃ | H | H |
| B-66 | CH=CH₂ | H | H | H | H |
| B-67 | H | CH=CH₂ | H | H | H |
| B-68 | H | H | CH=CH₂ | H | H |
| B-69 | CH=CH₂ | H | CH=CH₂ | H | H |
| B-70 | H | CH=CH₂ | CH=CH₂ | H | H |
| B-71 | CH₂CH=CH₂ | H | H | H | H |
| B-72 | H | CH₂CH=CH₂ | H | H | H |
| B-73 | H | H | CH₂CH=CH₂ | H | H |
| B-74 | CH₂CH=CH₂ | H | CH₂CH=CH₂ | H | H |
| B-75 | H | CH₂CH=CH₂ | CH₂CH=CH₂ | H | H |
| B-76 | C≡CH | H | H | H | H |
| B-77 | H | C≡CH | H | H | H |
| B-78 | H | H | C≡CH | H | H |
| B-79 | C≡CH | H | C≡CH | H | H |
| B-80 | H | C≡CH | C≡CH | H | H |
| B-81 | CH₂C≡CH | H | H | H | H |
| B-82 | H | CH₂C≡CH | H | H | H |
| B-83 | H | H | CH₂C≡CH | H | H |
| B-84 | CH₂C≡CH | H | CH₂C≡CH | H | H |
| B-85 | H | CH₂C≡CH | CH₂C≡CH | H | H |
| B-86 | OCH₃ | H | H | H | H |
| B-87 | H | OCH₃ | H | H | H |
| B-88 | H | H | OCH₃ | H | H |
| B-89 | OCH₃ | H | OCH₃ | H | H |
| B-90 | H | OCH₃ | OCH₃ | H | H |
| B-91 | OCH₂CH₃ | H | H | H | H |
| B-92 | H | OCH₂CH₃ | H | H | H |
| B-93 | H | H | OCH₂CH₃ | H | H |
| B-94 | OCH₂CH₃ | H | OCH₂CH₃ | H | H |
| B-95 | H | OCH₂CH₃ | OCH₂CH₃ | H | H |
| B-96 | OCH₂CH₂CH₃ | H | H | H | H |
| B-97 | H | OCH₂CH₂CH₃ | H | H | H |
| B-98 | H | H | OCH₂CH₂CH₃ | H | H |
| B-99 | OCH₂CH₂CH₃ | H | OCH₂CH₂CH₃ | H | H |
| B-100 | H | OCH₂CH₂CH₃ | OCH₂CH₂CH₃ | H | H |
| B-101 | O(CH₂)₃CH₃ | H | H | H | H |
| B-102 | H | O(CH₂)₃CH₃ | H | H | H |
| B-103 | H | H | O(CH₂)₃CH₃ | H | H |
| B-104 | O(CH₂)₃CH₃ | H | O(CH₂)₃CH₃ | H | H |
| B-105 | H | O(CH₂)₃CH₃ | O(CH₂)₃CH₃ | H | H |
| B-106 | O(CH₂)₄CH₃ | H | H | H | H |
| B-107 | H | O(CH₂)₄CH₃ | H | H | H |
| B-108 | H | H | O(CH₂)₄CH₃ | H | H |
| B-109 | O(CH₂)₄CH₃ | H | O(CH₂)₄CH₃ | H | H |
| B-110 | H | O(CH₂)₄CH₃ | O(CH₂)₄CH₃ | H | H |
| B-111 | O(CH₂)₅CH₃ | H | H | H | H |
| B-112 | H | O(CH₂)₅CH₃ | H | H | H |
| B-113 | H | H | O(CH₂)₅CH₃ | H | H |
| B-114 | O(CH₂)₅CH₃ | H | O(CH₂)₅CH₃ | H | H |
| B-115 | H | O(CH₂)₅CH₃ | O(CH₂)₅CH₃ | H | H |
| B-116 | QCH(CH₃)CH₂CH₃ | H | H | H | H |
| B-117 | H | OCH(CH₃)CH₂CH₃ | H | H | H |
| B-118 | H | H | OCH(CH₃)CH₂CH₃ | H | H |
| B-119 | OCH(CH₃)CH₂CH₃ | H | OCH(CH₃)CH₂CH₃ | H | H |
| B-120 | H | OCH(CH₃)CH₂CH₃ | OCH(CH₃)CH₂CH₃ | H | H |
| B-121 | OCH₂CH(CH₃)CH₂CH₃ | H | H | H | H |
| B-122 | H | OCH₂CH(CH₃)CH₂CH₃ | H | H | H |
| B-123 | H | H | OCH₂CH(CH₃)CH₂CH₃ | H | H |
| B-124 | OCH₂CH(CH₃)CH₂CH₃ | H | OCH₂CH(CH₃)CH₂CH₃ | H | H |
| B-125 | H | OCH₂CH(CH₃)CH₂CH₃ | OCH₂CH(CH₃)CH₂CH₃ | H | H |
| B-126 | OCH₂CH(CH₂CH₃)₂ | H | H | H | H |
| B-127 | H | OCH₂CH(CH₂CH₃)₂ | H | H | H |
| B-128 | H | H | OCH₂CH(CH₂CH₃)₂ | H | H |
| B-129 | OCH₂CH(CH₂CH₃)₂ | H | OCH₂CH(CH₂CH₃)₂ | H | H |
| B-130 | H | OCH₂CH(CH₂CH₃)₂ | OCH₂CH(CH₂CH₃)₂ | H | H |
| B-131 | OC(CH₃)₃ | H | H | H | H |
| B-132 | H | OC(CH₃)₃ | H | H | H |
| B-133 | H | H | OC(CH₃)₃ | H | H |
| B-134 | OC(CH₃)₃ | H | OC(CH₃)₃ | H | H |
| B-135 | H | OC(CH₃)₃ | OC(CH₃)₃ | H | H |
| B-136 | OCH₂C(CH₃)₃ | H | H | H | H |
| B-137 | H | OCH₂C(CH₃)₃ | H | H | H |
| B-138 | H | H | OCH₂C(CH₃)₃ | H | H |
| B-139 | OCH₂C(CH₃)₃ | H | OCH₂C(CH₃)₃ | H | H |
| B-140 | H | OCH₂C(CH₃)₃ | OCH₂C(CH₃)₃ | H | H |
| B-141 | CH₂CH₂OCH₂CH₃ | H | H | H | H |
| B-142 | H | CH₂CH₂OCH₂CH₃ | H | H | H |
| B-143 | H | H | CH₂CH₂OCH₂CH₃ | H | H |
| B-144 | CH₂CH₂OCH₂CH₃ | H | CH₂CH₂OCH₂CH₃ | H | H |
| B-145 | H | CH₂CH₂OCH₂CH₃ | CH₂CH₂OCH₂CH₃ | H | H |
| B-146 | CH₂CH₂O(CH₂)₂CH₃ | H | H | H | H |
| B-147 | H | CH₂CH₂O(CH₂)₂CH₃ | H | H | H |
| B-148 | H | H | CH₂CH₂O(CH₂)₂CH₃ | H | H |
| B-149 | CH₂CH₂O(CH₂)₂CH₃ | H | CH₂CH₂O(CH₂)₂CH₃ | H | H |
| B-150 | H | CH₂CH₂O(CH₂)₂CH₃ | CH₂CH₂O(CH₂)₂CH₃ | H | H |
| B-151 | CH₂CH₂CH₂OCH₂CH₃ | H | H | H | H |
| B-152 | H | CH₂CH₂CH₂OCH₂CH₃ | H | H | H |
| B-153 | H | H | CH₂CH₂CH₂OCH₂CH₃ | H | H |
| B-154 | CH₂CH₂CH₂OCH₂CH₃ | H | CH₂CH₂CH₂OCH₂CH₃ | H | H |
| B-155 | H | CH₂CH₂CH₂OCH₂CH₃ | CH₂CH₂CH₂OCH₂CH₃ | H | H |
| B-156 | (CH₂)₃O(CH₂)₂CH₃ | H | H | H | H |
| B-157 | H | (CH₂)₃O(CH₂)₂CH₃ | H | H | H |
| B-158 | H | H | (CH₂)₃O(CH₂)₂CH₃ | H | H |
| B-159 | (CH₂)₃O(CH₂)₂CH₃ | H | (CH₂)₃O(CH₂)₂CH₃ | H | H |
| B-160 | H | (CH₂)₃O(CH₂)₂CH₃ | (CH₂)₃O(CH₂)₂CH₃ | H | H |
| B-161 | (CH₂CH₂O)₂CH₂CH₃ | H | H | H | H |
| B-162 | H | (CH₂CH₂O)₂CH₂CH₃ | H | H | H |
| B-163 | H | H | (CH₂CH₂O)₂CH₂CH₃ | H | H |
| B-164 | (CH₂CH₂O)₂CH₂CH₃ | H | (CH₂CH₂O)₂CH₂CH₃ | H | H |
| B-165 | H | (CH₂CH₂O)₂CH₂CH₃ | (CH₂CH₂O)₂CH₂CH₃ | H | H |
| B-166 | (CH₂CH₂O)₂(CH₂)₂CH₃ | H | H | H | H |
| B-167 | H | (CH₂CH₂O)₂(CH₂)₂CH₃ | H | H | H |
| B-168 | H | H | (CH₂CH₂O)₂(CH₂)₂CH₃ | H | H |
| B-169 | (CH₂CH₂O)₂(CH₂)₂CH₃ | H | (CH₂CH₂O)₂(CH₂)₂CH₃ | H | H |
| B-170 | H | (CH₂CH₂O)₂(CH₂)₂CH₃ | (CH₂CH₂O)₂(CH₂)₂CH₃ | H | H |
| B-171 | (CH₂CH₂CH₂O)₂CH₂CH₃ | H | H | H | H |
| B-172 | H | (CH₂CH₂CH₂O)₂CH₂CH₃ | H | H | H |
| B-173 | H | H | (CH₂CH₂CH₂O)₂CH₂CH₃ | H | H |
| B-174 | (CH₂CH₂CH₂O)₂CH₂CH₃ | H | (CH₂CH₂CH₂O)₂CH₂CH₃ | H | H |
| B-175 | H | (CH₂CH₂CH₂O)₂CH₂CH₃ | (CH₂CH₂CH₂O)₂CH₂CH₃ | H | H |
| B-176 | ([CH₂]₃O)₂(CH₂)₂CH₃ | H | H | H | H |
| B-177 | H | ([CH₂]₃O)₂(CH₂)₂CH₃ | H | H | H |
| B-178 | H | H | ([CH₂]₃O)₂(CH₂)₂CH₃ | H | H |
| B-179 | ([CH₂]₃O)₂(CH₂)₂CH₃ | H | ([CH₂]₃O)₂(CH₂)₂CH₃ | H | H |
| B-180 | H | ([CH₂]₃O)₂(CH₂)₂CH₃ | ([CH₂]₃O)₂(CH₂)₂CH₃ | H | H |
| B-181 | (CH₂CH₂O)₃CH₂CH₃ | H | H | H | H |
| B-182 | H | (CH₂CH₂O)₃CH₂CH₃ | H | H | H |
| B-183 | H | H | (CH₂CH₂O)₃CH₂CH₃ | H | H |
| B-184 | (CH₂CH₂O)₃CH₂CH₃ | H | (CH₂CH₂O)₃CH₂CH₃ | H | H |
| B-185 | H | (CH₂CH₂O)₃CH₂CH₃ | (CH₂CH₂O)₃CH₂CH₃ | H | H |
| B-186 | (CH₂CH₂O)₃(CH₂)₂CH₃ | H | H | H | H |
| B-187 | H | (CH₂CH₂O)₃(CH₂)₂CH₃ | H | H | H |
| B-188 | H | H | (CH₂CH₂O)₃(CH₂)₂CH₃ | H | H |
| B-189 | (CH₂CH₂O)₃(CH₂)₂CH₃ | H | (CH₂CH₂O)₃(CH₂)₂CH₃ | H | H |
| B-190 | H | (CH₂CH₂O)₃(CH₂)₂CH₃ | (CH₂CH₂O)₃(CH₂)₂CH₃ | H | H |
| B-191 | (CH₂CH₂CH₂O)₃CH₂CH₃ | H | H | H | H |
| B-192 | H | (CH₂CH₂CH₂O)₃CH₂CH₃ | H | H | H |
| B-193 | H | H | (CH₂CH₂CH₂O)₃CH₂CH₃ | H | H |
| B-194 | (CH₂CH₂CH₂O)₃CH₂CH₃ | H | (CH₂CH₂CH₂O)₃CH₂CH₃ | H | H |
| B-195 | H | (CH₂CH₂CH₂O)₃CH₂CH₃ | (CH₂CH₂CH₂O)₃CH₂CH₃ | H | H |
| B-196 | ([CH₂]₃O)₃(CH₂)₂CH₃ | H | H | H | H |
| B-197 | H | ([CH₂]₃O)₃(CH₂)₂CH₃ | H | H | H |
| B-198 | H | H | ([CH₂]₃O)₃(CH₂)₂CH₃ | H | H |
| B-199 | ([CH₂]₃O)₃(CH₂)₂CH₃ | H | ([CH₂]₃O)₃(CH₂)₂CH₃ | H | H |
| B-200 | H | ([CH₂]₃O)₃(CH₂)₂CH₃ | ([CH₂]₃O)₃(CH₂)₂CH₃ | H | H |
| B-201 | C₆H₅ | H | H | H | H |
| B-202 | H | C₆H₅ | H | H | H |
| B-203 | H | H | C₆H₅ | H | H |
| B-204 | OC₆H₅ | H | H | H | H |
| B-205 | H | OC₆H₅ | H | H | H |
| B-206 | H | H | OC₆H₅ | H | H |
| B-207 | SC₆H₅ | H | H | H | H |
| B-208 | H | SC₆H₅ | H | H | H |
| B-209 | H | H | SC₆H₅ | H | H |
| B-210 | CH₂C₆H₅ | H | H | H | H |
| B-211 | H | CH₂C₆H₅ | H | H | H |
| B-212 | H | H | CH₂C₆H₅ | H | H |
| B-213 | OCH₂C₆H₅ | H | H | H | H |
| B-214 | H | OCH₂C₆H₅ | H | H | H |
| B-215 | H | H | OCH₂C₆H₅ | H | H |
| B-216 | SCH₂C₆H₅ | H | H | H | H |
| B-217 | H | SCH₂C₆H₅ | H | H | H |
| B-218 | H | H | SCH₂C₆H₅ | H | H |

Die Verbindungen I eignen sich als Fungizide. Sie zeichnen sich aus durch eine hervorragende Wirksamkeit gegen ein breites Spektrum von pflanzenpathogenen Pilzen aus der Klasse der *Ascomyceten, Deuteromyceten, Oomyceten* und *Basidiomyceten,* insbesondere aus der Klasse der *Oomyceten.* Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Speziell eignen sie sich zur Bekämpfung folgender Pflanzenkrankheiten:
- *Alternaria* Arten an Gemüse, Raps, Zuckerrüben und Obst und Reis,
- *Aphanomyces* Arten an Zuckerrüben und Gemüse,
- *Bipolaris-* und *Drechslera* Arten an Mais, Getreide, Reis und Rasen,
- *Blumeria graminis* (Echter Mehltau) an Getreide,
- *Botrytis cinerea* (Grauschimmel) an Erdbeeren, Gemüse, Blumen und Weinreben,
- *Bremia lactucae* an Salat,
- *CercosporaArten* an Mais, Sojabohnen, Reis und Zuckerrüben,
- *Cochliobolus* Arten an Mais, Getreide, Reis (z.B. *Cochliobolus sativus* an Getreide, *Cochliobolus miyabeanus* an Reis),
- *Colletotricum* Arten an Sojabohnen und Baumwolle,
- *Drechslera Arten* an Getreide und Mais,
- *Exserohilum* Arten an Mais,
- *Erysiphe cichoracearum* und *Sphaerotheca fuliginea* an Gurkengewächsen,
- *Fusarium* und *Verticillium* Arten an verschiedenen Pflanzen
- *Gaeumanomyces graminis* an Getreide
- *Gibberella* Arten an Getreide und Reis (z.B. *Gibberella fujikuroi* an Reis)
- *Grainstaining complex* an Reis,
- *Helminthosporium* Arten an Mais und Reis,
- *Michrodochium nivale* an Getreide,
- *Mycosphaerella* Arten an Getreide, Bananen und Erdnüssen,
- *Phakopsara pachyrhizi* und *Phakopsara meibomiae* an Sojabohnen,
- *Phomopsis* Arten an Sojabohnen und Sonnenblumen,
- *Phytophthora infestans* an Kartoffeln und Tomaten,
- *Plasmopara viticola* an Weinreben,
- *Podosphaera leucotricha* an Apfel,
- *Pseudocercosporella herpotrichoides* an Getreide,
- *Pseudoperonospora Arten* an Hopfen und Gurkengewächsen,
- *Puccinia* Arten an Getreide und Mais,
- *Pyrenophora* Arten an Getreide,
- *Pyricularia oryzae, Corticium sasakii, Sarocladium oryzae, S.attenuatum, Entyloma oryzae* an Reis,
- *Pyricularia grisea* an Rasen und Getreide,
- *Pythium spp.* an Rasen, Reis, Mais, Baumwolle, Raps, Sonnenblumen, Zuckerrüben, Gemüse und anderen Pflanzen,
- *Rhizoctonia-Arten* an Baumwolle, Reis, Kartoffeln, Rasen, Mais, Raps, Kartoffeln, Zuckerrüben, Gemüse und anderen Pflanzen,
- *Sclerotinia* Arten an Raps und Sonnenblumen,
- *Septoria tritici* und *Stagonospora nodorum* an Weizen,
- *Erysiphe* (syn. *Uncinula*) *necator* an Weinrebe,
- *Setospaeria* Arten an Mais und Rasen,
- *Sphacelotheca reilinia* an Mais,
- *Thievaliopsis* Arten an Sojabohnen und Baumwolle,
- *Tilletia Arten* an Getreide,
- *Ustilago* Arten an Getreide, Mais und Zuckerrübe und
- *Venturia* Arten (Schorf) an Apfel und Birne.

Insbesondere eignen sie sich zur Bekämpfung von Schadpilzen aus der Klasse der *Peronosporomycetes* (syn. *Oomyceten*), wie *Peronospora-*Arten, *Phytophthora-*Arten, *Plasmopara viticola* und *Pseudoperonospora-*Arten.

Die Verbindungen I eignen sich außerdem zur Bekämpfung von Schadpilzen im Materialschutz (z.B. Holz, Papier, Dispersionen für den Anstrich, Fasern bzw. Gewebe) und im Vorratsschutz. Im Holzschutz finden insbesondere folgende Schadpilze Beachtung: Ascomyceten wie *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomyceten wie *Coniophora* spp., *Coriolus* spp., *Gloeophyllum* spp., *Lentinus* spp., *Pleurotus* spp., *Poria* spp., *Serpula* spp. und *Tyromyces* spp., Deuteromyceten wie *Aspergillus* spp., *Cladosporium* spp., *Penicillium* spp., *Trichoderma* spp., *Alternaria* spp., *Paecilomycesspp.* und Zygomyceten wie *Mucorspp.,* darüber hinaus im Materialschutz folgende Hefepilze: *Candida* spp. und *Saccharomyces cerevisae.*

Die Verbindungen 1 werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung, z.B. durch Bestäuben, Beschichten oder Tränken von Saatgut, werden im allgemeinen Wirkstoffmengen von 1 bis 1000 g/100 kg, vorzugsweise 5 bis 100 g/100 kg Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel /Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Formulierungen für die Saatgutbehandlung können zusätzlich Bindemittel und/oder Geliermittel und gegebenenfalls Farbstoffe enthalten.

Bindemittel können zugesetzt werden, um Haftung der Wirkstoffe auf dem Saatgut nach der Behandlung zu erhöhen. Geeignete Bindemittel sind beispielsweise EO/PO Blockcopolymer-Tenside, aber auch Polyvinylalcohole, Ppolyvinylpyrrolidone, Polyacrylate, Polymethacrylate, Polybutene, Polyisobutylene, Polystyrole, Polyethylenamine, Polyethylenamide, Polyethylenimine (Lupasol®, Polymin®), Polyether, Polyurethane, Polyvinylacetate, Tylose und Copolymere aus diesen Polymeren. Ein geeignetes Geliermittel ist beispielsweise Carrageen (Satiagel®).

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Für die Saatgutbehandlung ergeben die betreffenden Formulierungen nach zwei- bis zehnfacher Verdünnung Wirkstoffkonzentrationen von 0,01 bis 60 Gew.-%, bevorzugt 0,1 bis 40 Gew.-% in den fertig verwendbaren Zubereitungen.

Beispiele für erfindungsgemäße Formulierungen sind:
1. Produkte zur Verdünnung in Wasser

### A Wasserlösliche Konzentrate (SL, LS)

10 Gew.-Teile einer erfindungsgemäßen Verbindung werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate (DC)

20 Gew.-Teile einer erfindungsgemäßen Verbindung werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

### C Emulgierbare Konzentrate (EC)

15 Gew.-Teile einer erfindungsgemäßen Verbindung werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen (EW, EO, ES)

25 Gew.-Teile einer erfindungsgemäßen Verbindung werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen (SC, OD, FS)

20 Gew.-Teile einer erfindungsgemäßen Verbindung werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-% .

### F Wasserdispergierbare und wasserlösliche Granulate (WG, SG)

50 Gew.-Teile einer erfindungsgemäßen Verbindung werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.%.

### G Wasserdispergierbare und wasserlösliche Pulver (WP, SP, SS, WS)

75 Gew.-Teile einer erfindungsgemäßen Verbindung werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen

In einer Kugelmühle werden 20 Gew.-Teile einer erfindungsgemäßen Verbindung, 10 Gew.-Teile Dispergiermittel, 1Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### I Stäube (DP, DS)

5 Gew.-Teile einer erfindungsgemäßen Verbindung werden fein gemahlen und mit 95 Gew.-Teilen feinteiligem Kaolin innig vermischt. Man erhält dadurch ein Stäubemittel mit 5 Gew.-% Wirkstoffgehalt.

### J Granulate (GR, FG, GG, MG)

0,5 Gew-Teile einer erfindungsgemäßen Verbindung werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### K ULV- Lösungen (UL)

10 Gew.-Teile einer erfindungsgemäßen Verbindung werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Für die Saatgutbehandlung werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Bevorzugt werden FS Formulierungen für die Saatgutbehandlung verwendet. Üblicherweise enthalten solche Formulierungen 1 bis 800 g/l Wirkstoff, 1 bis 200 g/l Tenside, 0 bis 200 g/l Frostschutzmittel, 0 bis 400 g/l Bindemittel, 0 bis 200 g/l Farbstoffe und Lösungsmittel, vorzugsweise Wasser.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergieroder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:100 bis 100:1, bevorzugt 1:10 bis 10:1 zugemischt werden.

Als Adjuvants in diesem Sinne kommen insbesondere in Frage: organisch modifizierte Polysiloxane, z.B. Break Thru S 240^{®}; Alkoholalkoxylate, z. B. Atplus 245®, Atplus MBA 1303^{®}, Plurafac LF 300^{®} und Lutensol ON 30^{®}; EO-PO-Blockpolymerisate, z. B. Pluronic RPE 2035^{®} und Genapol B^{®}; Alkoholethoxylate, z. B. Lutensol XP 80^{®}; und Natriumdioctylsulfosuccinat, z. B. Leophen RA^{®}.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen (I) bzw. der sie enthaltenden Mittel mit einem oder mehreren weiteren Wirkstoffen, insbesondere Fungiziden, kann in vielen Fällen das Wirkungsspektrum verbreitert oder Resistenzentwicklungen vorgebeugt werden. In vielen Fällen erhält man dabei synergistische Effekte.

Die folgende Liste von Fungiziden, mit denen die erfindungsgemäßen Verbindungen gemeinsam angewendet werden können, soll die Kombinationsmöglichkeiten erläutern, nicht aber einschränken:

### Strobilurine

Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethyl ester, 2-(ortho-(2,5-Dimethylphenyl-oxymethylen)phenyl)-3-methoxy-acrylsäuremethylester;

### Carbonsäureamide

- Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3,4-Dichlor-isothiazol-5-carbonsäure-(2-cyano-phenyl)-amid;
- Carbonsäuremorpholide: Dimethomorph, Flumorph;
- Benzoesäureamide: Flumetover, Fluopicolide (Picobenzamid), Zoxamide;
- Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;

### Azole

- Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazol, Myclobutanil, Penconazole, Propiconazole, Prothioconazole, Simeconazole, Tebuconazole, Tetraconazole, Triadimenol, Triadimefon, Triticonazole;
- Imidazole: Cyazofamid, Imazalil, Pefurazoate, Prochloraz, Triflumizole;
- Benzimidazole: Benomyl, Carbendazim, Fuberidazole, Thiabendazole;
- Sonstige: Ethaboxam, Etridiazole, Hymexazole;

### Stickstoffhaltige Heterocyclylverbindungen

- Pyridine: Fluazinam, Pyrifenox, 3-[5-(4-Chlor-phenyl)-2,3-dimethyl-isoxazolidin-3-yl]-pyridin;
- Pyrimidine: Bupirimate, Cyprodinil, Ferimzone, Fenarimol, Mepanipyrim, Nuarimol, Pyrimethanil;
- Piperazine: Triforine;
- Pyrrole: Fludioxonil, Fenpiclonil;
- Morpholine: Aldimorph, Dodemorph, Fenpropimorph, Tridemorph;
- Dicarboximide: Iprodione, Procymidone, Vinclozolin;
- sonstige: Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Pyroquilon, Quinoxyfen, Tricyclazole, 5-Chlor-7-(4-methyl-piperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluoro-2-methyl-indol-1-sulfonyl)-[1,2,4]triazol-1-sulfonsäuredimethylamid;

### Carbamate und Dithiocarbamate

- Dithiocarbamate: Ferbam, Mancozeb, Maneb, Metiram, Metam, Propineb, Thiram, Zineb, Ziram;
- Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluorphenyl)ester;

### Sonstige Fungizide

- Guanidine: Dodine, Iminoctadine, Guazatine;
- Antibiotika: Kasugamycin, Polyoxine, Streptomycin, Validamycin A;
- Organometallverbindungen: Fentin Salze;
- Schwefelhaltige Heterocyclylverbindungen: Isoprothiolane, Dithianon;
- Organophosphorverbindungen: Edifenphos, Fosetyl, Fosetyl-aluminium, Iprobenfos, Pyrazophos, Tolclofos-methyl, Phosphorige Säure und ihre Salze;
- Organochlorverbindungen: Thiophanate Methyl, Chlorothalonil, Dichlofluanid, Tolylfluanid, Flusulfamide, Phthalide, Hexachlorbenzene, Pencycuron, Quintozene;
- Nitrophenylderivate: Binapacryl, Dinocap, Dinobuton;
- Anorganische Wirkstoffe: Bordeaux Brühe, Kupferacetat, Kupferhydroxid, Kupferoxychlorid, basisches Kupfersulfat, Schwefel;
- Sonstige: Spiroxamine, Cyflufenamid, Cymoxanil, Metrafenone.

## Patentansprüche

1. Verwendung von 5-Amino-pyrazolen der Formel I 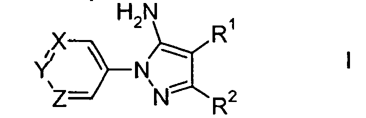 in der die Substituenten folgende Bedeutung haben:
R¹ Halogen, C₁-C₁₂-Alkyl, C₁-C₁₂-Halogenalkyl, C₃-C₆-Cycloalkyl, C₃-C₆-Halogencycloalkyl, C₂-C₁₂-Alkenyl, C₂-C₁₂-Halogenalkenyl, C₂-C₁₂-Alkinyl, C₂-C₁₂-Alkoxyalkyl, C₂-C₁₂-Halogenalkoxyalkyl, C₂-C₁₂-Alkoxyhalogenalkyl, C₂-C₁₂-Cyanoalkyl, C₁-C₁₂-Alkyl-C₁-C₁₂-alkoxyimino, Phenyl, Benzyloxy-C₁-C₁₂-alkyl, oder ein fünf- oder sechsgliedriger gesättigter, partiell ungesättigter oder aromatischer Heterocyclus, enthaltend ein bis vier Heteroatome aus der Gruppe O, N oder S, welcher entweder direkt oder über eine C₁-C₄-Alkylengruppe gebunden sind;
R² Halogen, Cyano, C₁-C₁₂-Alkyl, C₁-C₁₂-Halogenalkyl, C₂-C₁₂-Alkenyl, C₂-C₁₂-Alkinyl, C₃-C₈-Cycloalkyl, C₁-C₁₂-Alkoxy, C₁-C₁₂-Alkoxy-C₁-C₁₂-alkyl, C₁-C₁₂-Alkyl-C₁-C₁₂-alkoxyimino, Phenyl, Benzyloxy-C₁-C₁₂-alkyl, oder ein fünf- oder sechsgliedriger gesättigter, partiell ungesättigter oder aromatischer Heterocyclus, enthaltend ein bis vier Heteroatome aus der Gruppe O, N oder S, welcher entweder direkt oder über eine C₁-C₄-Alkylengruppe gebunden sind;
X, Y, Z X oder Y oder X und Z oder Y und Z für N und die jeweiligen anderen Variablen für C-R3;
R³ Wasserstoff oder eine der bei R^{a} genannten Gruppen;
die Gruppen R¹, R² und R³ können unabhängig voneinander unsubstituiert oder durch eine bis fünf Gruppen R^{a} substituiert sein:
R^{a} Halogen, Cyano, Hydroxy, Mercapto, C₁-C₁₀-Alkyl, C₁-C₁₀-Halogen-alkyl, C₃-C₈-Cycloalkyl, C₂-C₁₀-Alkenyl, C₂-C₁₀-Alkinyl, C₁-C₆-Alkoxy, C₁-C₆-Alkylthio, C₁-C₆-Alkoxy-C₁-C₆-alkyl, NR^{A}R^{B}, Phenyl, Phenoxy, Phenylthio, C₁-C₆-Alkyl-phenyl, C₁-C₆-Alkoxy-phenyl, C₁-C₆-Alkylthiophenyl;
R^{A}, R^{B} Wasserstoff und C₁-C₆-Alkyl;
wobei die cyclischen Gruppen in R^{a} durch eine bis vier Gruppen R^{b} substituiert sein können:
R^{b} Halogen, Cyano, Hydroxy, Mercapto, C₁-C₁₀-Alkyl, C₁-C₁₀-Halogenalkyl, C₂-C₁₀-Alkenyl, C₂-C₁₀-Alkinyl und C₁-C₆-Alkoxy;
zur Bekämpfung von pflanzenpathogenen Schadpilzen.

2. Verbindungen der Formel I gemäß Anspruch 1.

3. Verbindungen der Formel I gemäß Anspruch 1, worin
R¹ für C₁-C₁₂-Alkyl, C₂-C₁₂-Alkoxyalkyl, Phenyl oder Benzyl, wobei die Ringsysteme durch eine bis fünf Gruppen R^{a} substituiert sein können; und
R² für C₁-C₅-Alkyl, C₁-C₅-Alkoxy-C₁-C₅-alkyl stehen.

4. Verbindungen der Formel I gemäß Anspruch 1, worin R¹ für n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 1-Methylhexyl, n-Octyl, 1-Methylheptyl, n-Nonyl, 1-Methyloctyl, 3,5,5-Trimethylhexyl, n-Decyl, 1-Methylnonyl, n-Undecyl, 1-Methyldecyl, n-Dodecyl und 1-Methylundecyl steht.

5. Verfahren zur Herstellung von Verbindungen der Formel I gemäß einem der Ansprüche 2 bis 4, in denen R² für Alkyl steht, **dadurch gekennzeichnet, dass** man α-Ketonitrile der Formel II 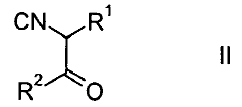 mit Hydrazinen der Formel III 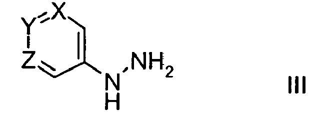 umsetzt.

6. Verfahren zur Herstellung von Verbindungen der Formel I gemäß einem der Ansprüche 2 bis 4, in denen R² für Alkyl steht, **dadurch gekennzeichnet, daß** man β-Ketoester der Formel IIa 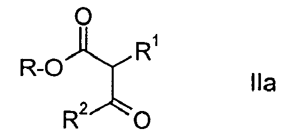 in der R C₁-C₄-Alkyl bedeutet, welche durch Halogenierungsmittel [HAL]in die Säurehalogenide der Formel IIb 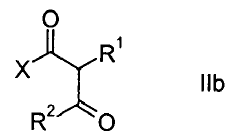 in der X für Halogen steht, überführt werden, welche mit Aminen der Formel HNR₂, in der R die o. g. Bedeutung hat, zu Amiden der Formel IIc 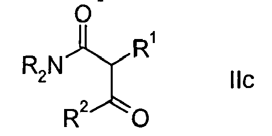 welche mit Hydrazinen der Formel III gemäß Anspruch 5 umgesetzt werden.

7. Fungizides Mittel, enthaltend einen festen oder flüssigen Träger und eine Verbindung der Formel I gemäß Anspruch 2.

8. Mittel gemäß Anspruch 7, enthaltend zusätzlich einen weiteren Wirkstoff.

9. Saatgut, enthaltend eine Verbindung der Formel I gemäß Anspruch 1 in einer Menge von 1 bis 1000 g pro 100 kg.

10. Verfahren zur Bekämpfung von pflanzenpathogenen Schadpilzen, **dadurch gekennzeichnet, daß** man die Pilze, oder die vor Pilzbefall zu schützenden Materialien, Pflanzen, den Boden oder Saatgüter mit einer wirksamen Menge einer Verbindung der Formel I gemäß Anspruch 1 behandelt.
